# EUROPÄISCHE PATENTANMELDUNG

(11) **EP 2 742 879 A2**
(43) Veröffentlichungstag der Anmeldung: **18.06.2014**
(21) Anmeldenummer: 13197689.6
(22) Anmeldetag: 17.12.2013
(51) Int. Cl.: A61B 17/221

(54) **Medizinisches Instrument für intravaskuläre Eingriffe und Herstellungsverfahren**

(30) Priorität: 17.12.2012 DE 102012112365; 29.11.2013 DE 102013113263
(71) Anmelder: Acandis GmbH & Co. KG, 76327 Pfinztal (DE)
(72) Erfinder: Nagl, Frank, 76137 Karlsruhe (DE)
(74) Vertreter: Kilchert, Jochen

(57) **Zusammenfassung**

Die Erfindung betrifft ein medizinisches Instrument für intravaskuläre Eingriffe mit einem zylindrischen Element (11), das durch einen Katheter in einen Körperhohlraum einführbar ist, wobei das zylindrische Element (11) einen distalen Endabschnitt (12) mit einem radial expandierbaren Funktionsabschnitt (13) und einem durchmesserstabilen, zylindrischen Führungsabschnitt (14) aufweist, der proximal zum Funktionsabschnitt (13) angeordnet ist. Die Erfindung zeichnet sich dadurch aus, dass der distale Endabschnitt (12) zur Bildung des Funktionsabschnitts (13) strukturiert ist derart, dass der Funktionsabschnitt (13) wenigstens einen Steg (27) aufweist, der eine einstückige Verlängerung des Führungsabschnitts (14) bildet. Die Erfindung betrifft ferner ein Herstellungsverfahren für ein medizinisches Instrument (10).

## Beschreibung

Die Erfindung betrifft ein medizinisches Instrument für intravaskuläre Eingriffe sowie ein Verfahren zum Herstellen eines medizinischen Instruments. Ein Instrument mit den Merkmalen des Oberbegriffs des Anspruchs 1 ist beispielsweise aus US 6,125,685 bekannt.

Aus der Praxis bekannte Vorrichtungen weisen ein Funktionselement auf, das im Gebrauch die Rekanalisationsfunktion übernimmt. Meist sind die Funktionselemente radial expandierbar, so dass sie über einen relativ kleinlumigen Katheter in ein Blutgefäß eingeführt und darin zu ihrer wirksamen Größe aufgespannt werden können. Zum Einführen des Funktionselements dient ein an sich bekannter Transportdraht, der mit dem Funktionselement verbunden ist. Die Verbindung zwischen dem Funktionselement und dem Transportdraht wird üblicherweise durch Schweißen, Löten, Kleben, Pressen oder dergleichen Verbindungstechniken hergestellt. Gerade aufgrund der relativ kleinen Dimensionen der vorgenannten Instrumente, ergeben sich besondere Herausforderungen bei deren Herstellung. Insbesondere ist eine besonders hohe Präzision erforderlich, um das Funktionselement mit dem Transportdraht zu verbinden. Überdies sind Qualitätssicherungsmaßnahmen, insbesondere die Überprüfung der Verbindung zwischen dem Funktionselement und dem Transportdraht, besonders aufwändig. Dies gilt vor allem für Schweißverbindungen, die bei den sehr kleinen Dimensionen intravaskulärer Eingriffsinstrumente schwer zu überwachen sind. Die Überprüfung, ob eine Schweißverbindung richtig gesetzt ist, ist wegen der kleinen Dimensionen besonders zeit- und kostenintensiv und das Restrisiko, dass die Schweißverbindung den erforderlichen Qualitätsstandards nicht genügt, entsprechend hoch.

Die Aufgabe der Erfindung besteht darin, ein medizinisches Instrument für intravaskuläre Eingriffe anzugeben, das eine einfache Herstellung erlaubt und eine hohe Stabilität aufweist. Ferner ist es Aufgabe der Erfindung, ein Verfahren zum Herstellen eines medizinischen Instruments für intravaskuläre Eingriffe anzugeben.

Erfindungsgemäß wird diese Aufgabe im Hinblick auf das Instrument durch den Gegenstand des Patentanspruchs 1 und im Hinblick auf das Verfahren durch den Gegenstand des Patentanspruchs 13 gelöst.

Die Erfindung beruht auf dem Gedanken, ein medizinisches Instrument für intravaskuläre Eingriffe mit einem zylindrischen Element anzugeben, das durch einen Katheter in einen Körperhohlraum einführbar ist, wobei das zylindrische Element einen distalen Endabschnitt mit einem radial expandierbaren Funktionsabschnitt und einem durchmesserstabilen, zylindrischen Führungsabschnitt aufweist, der proximal zum Funktionsabschnitt angeordnet ist. Der distale Endabschnitt ist zur Bildung des Funktionsabschnitts derart strukturiert, dass der Funktionsabschnitt wenigstens einen Steg aufweist, der eine einstückige Verlängerung des Führungsabschnitts bildet.

Der Funktionsabschnitt eines erfindungsgemäßen Instruments kann beispielsweise eine kathetergeführte Rekanalisationsvorrichtung wie ein Mikrodrahtretriever, Fangkorb, insbesondere für die Thrombektomie, Filter oder dergleichen sein. Der Funktionsabschnitt eines derartigen Instruments kann auch ein Manipulationsorgan, wie ein Greifer, sein.

Der Führungsabschnitt kann einerseits einen Transportdraht bilden, der im Gebrauch vom Funktionsabschnitt bis zum Anwender verläuft, so dass dieser den Funktionsabschnitt betätigen kann, bspw. durch Vorschieben des Funktionsabschnitts im Katheter, durch Freisetzen des Funktionsabschnitts, durch Wiedereinziehen des Funktionsabschnitts und/oder durch Bewegen des Funktionsabschnitts im Gefäß. Der Transportdraht und der Funktionsabschnitt bilden ein einstückiges Bauteil aus demselben Material.

Andererseits kann der Führungsabschnitt ein Transportdrahtsegment bilden, das kürzer als ein Transportdraht ist. Das Transportdrahtsegment ist im Gebrauch vom Anwender distal beabstandet im Katheter oder im Gefäß angeordnet. Zur Betätigung des Funktionsabschnittes ist das Transportdrahtsegment mit einem Transportdraht verbindbar oder verbunden, der im Gebrauch vom Transportdrahtsegment bis zum Anwender verläuft, so dass dieser den Funktionsabschnitt, wie vorstehend erläutert, bewegen kann. Das Transportdrahtsegment und der Funktionsabschnitt bilden ein einstückiges Bauteil aus demselben Material. Zusammen mit dem Transportdraht bilden das Transportdrahtsegment und der Funktionsabschnitt ein zweiteiliges Bauteil.

Anstelle der bisher bekannten Herstellung medizinischer Instrumente für intravaskuläre Eingriffe aus zwei separaten Bauteilen, nämlich dem Funktionselement und dem Transportdraht bzw. Führungsdraht, wird erfindungsgemäß das gesamte Instrument, insbesondere der Funktionsabschnitt und der Führungsabschnitt einstückig hergestellt. Die Herstellung des Funktionsabschnitts und der Führungsabschnitts erfolgt aus ein und demselben Halbzeug bzw. Ausgangsmaterial, bspw. aus einem Volldraht oder einem Hohldraht.Durch die einstückige Herstellung des Instruments ist der distale Endabschnitt derart strukturiert, dass der radial expandierbare Funktionsabschnitt mit wenigstens einem Steg gebildet ist. Der Steg bildet eine einstückige Verlängerung des Führungsabschnittes. Zwischen dem Führungsabschnitt und dem Funktionsabschnitt besteht somit eine stoffschlüssige Verbindung aus demselben Material ohne Gefügeveränderung - bspw. im Unterschied zu einer Schweißverbindung. Die Verbindung zwischen dem Führungsabschnitt und dem Funktionsabschnitt ist daher stabil und sicher. Außerdem können durch die derzeit möglichen Abtragverfahren unterschiedliche Stegformen und/oder Stegbreiten und/oder Stegquerschnittsprofile und/oder Stegverläufe und/oder Anzahlen von Stegen gefertigt werden, die verschiedene Funktionalitäten, wie vorstehend ausgeführt, aufweisen. Das Instrument ermöglicht daher ein breites Anwendungsspektrum.

Unter einem Steg wird ein längliches Element verstanden, das im radial expandierten Zustand in Längsrichtung insgesamt oder zumindest abschnittsweise frei von anderen Bauteilen ist. Der Steg ist im radial expandierten Zustand von anderen Elementen des Funktionsabschnittes insgesamt oder zumindest abschnittsweise beabstandet. Das proximale Ende des Steges ist dabei einstückig mit dem Führungsabschnitt verbunden.

Der zylindrische Führungsabschnitt ist durchmesserstabil und kann somit nicht radial expandieren, wenn dieser aus dem Katheter entlassen wird. Die Form und Durchmesserstabilität des Führungsabschnittes ergibt sich aus der einstückigen Herstellung des zylindrischen Elementes, wobei die Außenkontur des Führungsabschnittes der Außenkontur des Ausgangsmaterials vor der Bearbeitung entspricht.

Bevorzugte Ausführungen der Erfindung sind in den Unteransprüchen angegeben.

Der Funktionsabschnitt kann eine zumindest abschnittsweise röhrchenförmige Wandung aufweisen, die durch den Steg, insbesondere durch mehrere Stege gebildet ist. Die radial expandierbare Wandung ist somit einstückig mit dem Führungsabschnitt verbunden. Die Wandung kann durch eine helixförmige bzw. schraubenförmige Anordnung eines einzigen Steges gebildet sein. Mit wenigstens zwei Stegen ist die Bildung einer gitterförmigen Wandung möglich.

Diese Ausführung ist für ein einstückig mit dem Führungsabschnitt verbundenes Rekanalisationsgerät besonders geeignet und hat den Vorteil, dass durch die stabile Verbindung zwischen dem Rekanalisationsgerät und dem Führungsabschnitt ein sicheres Wiedereinziehen des Rekanalisationsgeräts in den Katheter erreicht wird.

Der Funktionsabschnitt kann ein Manipulationsorgan bilden, wobei der Steg, insbesondere mehrere Stege zur Manipulation eines Implantats angepasst sind. Die einstückige Verbindung zwischen dem Manipulationsorgan und dem Führungsabschnitt ist relativ steif, so dass Bewegungen und Kräfte bzw. Momente vom Führungsabschnitt auf das Manipulationsorgan sicher und präzise übertragbar sind.

Vorzugsweise ist das gesamte zylindrische Element, insbesondere das gesamte Instrument, einstückig aus demselben Material ausgebildet. Somit wird aus einem einzigen Ausgangsmaterial, dem durch einen Katheter führbaren zylindrischen Element, ein Funktionsabschnitt und ein Führungsabschnitt gebildet. Dabei ist es ausreichend, ausschließlich den distalen Endabschnitt des zylindrischen Elements verfahrenstechnisch zu bearbeiten, um den Funktionsabschnitt zu bilden. Der Führungsabschnitt kann durch einen nicht-expandierbaren Abschnitt des zylindrischen Elements gebildet sein, der sich an den Funktionsabschnitt in proximaler Richtung anschließt. Das zylindrische Element kann konkret ein Drahtelement oder Draht, insbesondere ein Volldraht oder Hohldraht, sein, der im endgefertigten Zustand zumindest am distalen Ende zur Bildung des Funktionsabschnittes strukturiert ist.

Die Strukturierung des distalen Endabschnitts erfolgt vorzugsweise durch einen materialabtragenden Bearbeitungsschritt. Das bedeutet, dass bereichsweise Material des zylindrischen Elements entfernt wird. Vorzugsweise wird das zylindrische Element in Querschnittsrichtung, insbesondere in radialer Richtung, vollständig durchschnitten. Der materialabtragende Bearbeitungsschritt erfolgt derart, dass ein radial expandierbarer Funktionsabschnitt gebildet ist. Es wird also Material des zylindrischen Elements abgetragen bzw. entfernt, so dass eine Struktur gebildet wird, die sich radial aufweiten kann. Der Funktionsabschnitt kann einen radial komprimierten Zustand aufweisen, bei dem der Außendurchmesser des Funktionsabschnitts im Wesentlichen dem Außendurchmesser des zylindrischen Elements bzw. des Führungsabschnitts des zylindrischen Elements entspricht. Ferner weist der Funktionsabschnitt einen expandierten Zustand auf, in dem der Funktionsabschnitt einen Querschnittsdurchmesser, insbesondere einen Außendurchmesser, aufweist, der größer als der Außendurchmesser des zylindrischen Elements, insbesondere des Führungsabschnitts des zylindrischen Elements, ist.

In einer weiteren bevorzugten Ausgestaltung der Erfindung ist vorgesehen, dass in dem Endabschnitt über den Umfang verteilt mehrere Längsschnitte angeordnet sind. Der materialabtragende Bearbeitungsschritt kann also derart ausgestaltet sein, dass in das zylindrische Element Schnitte eingebracht werden, die sich in Längsrichtung des zylindrischen Elements erstrecken. Durch das Einbringen von Längsschnitten wird erreicht, dass der gebildete Funktionsabschnitt maßgeblich in radialer Richtung expandierbar ist. Die Längsschnitte erfolgen zur Bildung der Stege. Dabei wird vorzugsweise als Halbzeug bzw. Ausgangsmaterial ein Hohldraht verwendet. Die Stegdicke ergibt sich aus der Wandstärke des Hohldrahtes. Die Stegbreite ergibt sich aus dem Abstand der in Umfangsrichtung unmittelbar benachbarten Längsschnitte. Im Extremfall kann auf diese Weise ein einziger Steg aus der Wandung geformt, insbesondere lasergeschnitten werden. Die Verwendung eines Hohldrahtes wird für die Herstellung einer Gitterstruktur aus mehrere Stegen bevorzugt. Die Herstellung von Einzelstegen, die frei voneinander bewegbar sind, bspw. als Greiffinger eines Manipulationsorgans, kann aus einem Hohldraht oder einem Volldraht erfolgen.

Die radiale Expandierbarkeit des Steges, also die Fähigkeit des Steges, sich radial nach außen zu bewegen, kann bspw. durch Verformen erreicht werden, wobei der Steg im komprimierten Zustand vorgespannt ist und durch Aufheben der Kompressionskraft, bspw. durch Freisetzen aus dem Katheter, sich radial nach Außen bewegt, bspw. in den Ruhezustand oder in den implantierten Zustand. Bei einem Formgedächtniswerkstoff, wie Nitinol, kann der Steg in an sich bekannter Weise vorkonditioniert sein. Die vorstehend beschriebene radiale Expandierbarkeit des Steges wird im Zusammenhang mit allen Ausführungen offenbart.

Es kann vorgesehen sein, dass die Längsschnitte in Längsrichtung zueinander versetzt angeordnet sind, so dass der Funktionsabschnitt eine netzartige oder gitterartige Struktur aufweist. Insbesondere kann vorgesehen sein, dass mehrere Längsschnitte zueinander fluchtend und in Längsrichtung benachbart zueinander angeordnet sind. Die zueinander fluchtenden Längsschnitte bilden eine Längsschnittreihe. In Umfangsrichtung benachbart kann eine weitere Längsschnittreihe vorgesehen sein, wobei die Längsschnitte der weiteren Längsschnittreihe in Längsrichtung zu den Längsschnitten der ersten Längsschnittreihe versetzt sind. Insbesondere ist ein Versatz um eine halbe Längsschnittlänge bevorzugt. Auf dieses Weise wird ein Funktionsabschnitt bereitgestellt, der zumindest teilweise eine netzartige Struktur, insbesondere eine Gitterstruktur, aufweist. Der Funktionsabschnitt ist somit zumindest teilweise stentartig ausgebildet. Wenigstens im stentartigen Teil des Funktionsabschnitts kann der Funktionsabschnitt eine zylinderförmige Außenkontur aufweisen, insbesondere hohlzylinderförmig ausgebildet sein.

Die Längsschnitte oder zumindest ein Teil der Längsschnitte können sich bis zum distalen Ende des zylindrischen Elements, insbesondere des Endabschnitts, erstrecken, so dass der Funktionsabschnitt eine greiferartige Struktur aufweist. Der Endabschnitt kann also derart strukturiert sein, dass das distale Ende des zylindrischen Elements, insbesondere eine distale Endfläche des zylindrischen Elements, in der Vorderansicht im Wesentlichen einer Vorderansicht eines Mehrbackenfutters, beispielsweise eines Dreibackenfutters, ähnelt. Das zylindrischen Element ist somit vom distalen Ende aus in wenigstens zwei, vorzugsweise drei oder mehr, Greifarme aufgespalten, die den Funktionsabschnitt bilden. Verfahrenstechnisch kann die Herstellung derartiger Greifarme im Rahmen der Erfindung durch das Einbringen von Längsschnitten in den Endabschnitt des zylindrischen Elements erfolgen, die sich bis zum distalen Ende des zylindrischen Elements erstrecken. Vorzugsweise sind über den Umfang des zylindrischen Elements verteilt mehrere, insbesondere drei oder mehr, Längsschnitte angeordnet, die bis zum distalen Ende des zylindrischen Elements verlaufen.

Generell können der Steg bzw. die Stege jeweils ein distales freies Ende aufweisen, das vom Führungsabschnitt beabstandet ist.

Im Rahmen der Erfindung kann ferner vorgesehen sein, dass der Führungsabschnitt eine Oberflächenstrukturierung und/oder eine Perforation aufweist. Entlang des Führungsabschnittes können mehrere in Umfangsrichtung orientierte Einschnitte angeordnet sein, so dass die Verlängerung in einem distalen Bereich eine höhere Biegeflexibilität als in einem proximalen Bereich aufweist. Konkret können zumindest im Führungsabschnitt ringförmig und/oder helixförmig umlaufende Materialschwächungen und/oder Materialdurchbrüche angeordnet sein.

Generell kann das zylindrische Element, das durch den Funktionsabschnitt und den Führungsabschnitt gebildet ist, im Führungsabschnitt bereichsweise einen reduzierten Querschnitt aufweisen. Vorzugsweise ist die Oberflächenstrukturierung und/oder Perforation in einem distalen Bereich des Führungsabschnitts angeordnet, die sich unmittelbar an den Funktionsabschnitt anschließt. Die Oberflächenstrukturierung und/oder Perforation kann sich über den gesamten Umfang des zylindrischen Elements erstrecken. Insbesondere kann die Oberflächenstrukturierung und/oder die Perforation durch Einschnitte gebildet sein, die sich in Umfangsrichtung des Führungsabschnittes erstrecken, d.h. in Umfangsrichtung orientiert sind. Die Einschnitte sind vorzugsweise derart orientiert bzw. angeordnet, dass der Führungsabschnitt in einem distalen Bereich eine höhere Biegeflexibilität als in einem proximalen Bereich aufweist.

Die Einschnitte können im Wesentlichen ringförmig ausgebildet sein. Es ist auch möglich, dass die Einschnitte eine Länge aufweisen, die kleiner als der Umfang des zylindrischen Elements ist. Die Einschnitte können sich über einen Teil des Umfangs des zylindrischen Elements erstrecken. Im Allgemeinen sind mehrere Einschnitte vorgesehen, die in Längsrichtung bzw. längsaxialer Richtung des zylindrischen Elements, insbesondere des Führungsabschnittes benachbart und zueinander beabstandet angeordnet sind. Die Abstände zwischen den Einschnitten können unterschiedlich sein. Ferner kann vorgesehen sein, dass die in Längsrichtung benachbarten Einschnitte in Umfangsrichtung zueinander versetzt angeordnet sind. In allen Fällen ist es bevorzugt, wenn die Einschnitte in einem distalen Bereich der Verlängerung angeordnet sind, der sich direkt an den Funktionsabschnitt anschließt. Dadurch wird die Biegeflexibilität des zylindrischen Elements, insbesondere des Führungsabschnitts, bereichsweise erhöht. Dies erleichtert die Zufuhr des Instruments in Körpergefäße, die stark gekrümmt sind bzw. die über stark gekrümmte Gefäße zugänglich sind. Das Instrument kann einer Gefäßkrümmung aufgrund der bereichsweise erhöhten Biegeflexibilität gut folgen.

Vorzugsweise sind die Einschnitte entlang des Führungsabschnitts derart gestaltet, dass sich die Biegeflexibilität entlang des Führungsabschnitts ändert, insbesondere in distaler Richtung zunimmt. Dies kann dadurch erreicht werden, dass sich die Breite und/oder Tiefe der Einschnitte vom distalen Bereich zum proximalen Bereich der Verlängerung reduziert. Alternativ oder zusätzlich kann sich der Abstand der Einschnitte vom distalen Bereich zum proximalen Bereich der Verlängerung erhöhen. Beide vorgenannten Maßnahmen ermöglichen eine Steigerung der Biegeflexibilität in distaler Richtung der Verlängerung. So kann erreicht werden, dass die Verlängerung im proximalen Bereich ausreichend steif ist, um ein Vorschieben des zylindrischen Elements zu stabilisieren. Mit dem biegeflexibleren, distalen Bereich der Verlängerung wird die Manövrierfähigkeit des zylindrischen Elements bzw. allgemein des medizinischen Instruments in Körperhohlorganen verbessert.

Die Einschnitte können im Allgemeinen ringförmig oder helixförmig umlaufende Materialschwächungen oder Materialdurchbrüche umfassen. Die Materialschwächungen können beispielsweise als Nuten ausgebildet sein bzw. im weitesten Sinne eine Oberflächenstrukturierung bilden. Die Einschnitte können auch als Materialdurchbrüche ausgebildet sein, also das Material der Verlängerung vollständig durchdringen, d.h. den Führungsabschnitt perforieren.

In einer bevorzugten Ausgestaltung des medizinischen Instruments sind die Einschnitte, insbesondere die Materialdurchbrüche, zumindest bereichsweise um eine Längsachse des Führungsabschnitts verdreht angeordnet. Auf diese Weise können Vorzugsrichtungen für die Biegeflexibilität eingestellt werden. Konkret bedeutet das, dass der Führungsabschnitt mehrere Einschnitte aufweist, wobei ein oder mehrere Einschnitte zu einem Einschnittbereich zusammengefasst sein können. Unterschiedliche Einschnittbereiche können im Hinblick auf ihre Orientierung bezüglich der Längsachse des Führungsabschnitts zueinander verdreht angeordnet sein. Insbesondere kann ein erster Einschnittbereich und ein benachbarter zweiter Einschnittbereich vorgesehen sein, wobei die Einschnitte des ersten Einschnittbereichs und die Einschnitte des zweiten Einschnittbereichs zueinander um 90 Grad um die Längsachse des Führungsabschnitts verdreht angeordnet sind. So weist der erste Einschnittbereich eine erhöhte Biegeflexibilität in einer ersten queraxialen Vorzugsrichtung und der zweite Einschnittbereich eine Biegeflexibilität in einer zweiten, senkrecht zur ersten Vorzugsrichtung angeordneten queraxialen Vorzugsrichtung auf.

Allgemein wird offenbart, dass das zylindrische Element durch einen Volldraht oder einen Hohldraht gebildet sein kann. Es ist bevorzugt, wenn das medizinische Instrument einen Funktionsabschnitt und einen Führungsabschnitt aufweist, die aus ein und demselben Ausgangsmaterial, nämlich ein und demselben zylindrischen Element, gebildet sind. Bei Verwendung eines Hohldrahts können die für die bereichsweise erhöhte Biegeflexibilität eingebrachten Einschnitte eine Einschnitttiefe aufweisen, die der Wandstärke des Hohldrahts entspricht. Mit anderen Worten können die Einschnitte die Hohldrahtwandung vollständig durchbrechen, so dass die Einschnitte im Wesentlichen Materialdruchbrüche in der Wandung des Hohldrahts bilden. Die Einschnitte bilden in diesem Fall eine Perforation. Die Verwendung eines Hohldrahts als Ausgangsmaterial für die Herstellung des medizinischen Instruments ermöglicht insbesondere die Ausbildung von Funktionsabschnitten, die eine netzartige Struktur aufweisen. Funktionsabschnitte, die Greifarme umfassen bzw. bilden, können gut aus einem Volldraht oder einem Hohldraht geschnitten werden.

Bei einer weiteren bevorzugten Ausführungsform des erfindungsgemäßen medizinischen Instruments ist vorgesehen, dass der Führungsabschnitt ein Transportelement, insbesondere einen Transportdraht oder ein Transportdrahtsegment bildet. Das Transportdrahtsegment kann mit einem Transportdraht verbunden oder verbindbar sein. Grundsätzlich erstreckt sich das Transportelement bis zu einem proximalen Ende des Instruments. Im Gebrauch ist das proximale Ende des Transportelements, insbesondere des Transportdrahts, vorzugsweise außerhalb des Körpers angeordnet, so dass ein Anwender des das proximale Ende greifen kann. Wenn der Führungsabschnitt den Transportdraht bildet, ist das gesamte medizinische Instrument einstückig ausgebildet, insbesondere aus demselben Ausgangsmaterial hergestellt. Bei der alternativen Variante, bei der der Führungsabschnitt ein Transportdrahtsegment bildet, das mit dem Transportdraht verbunden oder verbindbar ist, bildet der Transportdraht ein separates Bauteil, das mit dem zylindrischen Element zu einem medizinischen Instrument zusammengefügt ist. Die Verbindung zwischen dem gesonderten Transportdraht und dem zylindrischem Element, insbesondere dem Führungsabschnitt, insbesondere dem Transportdrahtsegment, kann durch Schweißen, Löten, Kleben oder eine lösbare Verbindungstechnik, beispielsweise Schrauben, erfolgen.

Das Transportelement kann wenigstens ein Flexionselement aufweisen, das zur kontinuierlichen oder schrittweisen Steigerung der Biegeflexibilität angepasst ist. Das Flexionselement ist vorzugsweise zwischen dem proximalen Ende des Instruments, insbesondere des Transportelements, und dem zylindrischen Element angeordnet. Wenn der Führungsabschnitt den gesamten Transportdraht bildet, ist das Flexionselement zwischen dem proximalen Ende des Instruments und dem Funktionsabschnitt angeordnet. Wenn der Führungsabschnitt das Transportdrahtsegment bildet, ist das Flexionselement zwischen dem proximalen Ende des Instruments und dem Transportdrahtsegment angeordnet. Das Flexionselement kann beispielsweise durch eine Feder oder einen Coil gebildet sein, wobei die Feder oder der Coil eine in distale Richtung des Flexionselements bzw. des Transportelements sich erhöhende Steigung aufweist. Generell wird die Feder oder der Coil also durch einen helixförmig um die Längsachse des Transportelements gewundenen Draht gebildet, wobei sich der Abstand der einzelnen Windungen des helixförmigen Drahtes in distale Richtung des Transportelements, d.h. in Richtung des Funktionsabschnitts, erhöht. So weist das Flexionselement am proximalen Ende eine höhere Steifigkeit als am distalen Ende auf. Mit anderen Worten ist das Flexionselement am distalen Ende biegsamer als am proximalen Ende. Anstelle einer Feder oder eines Coils kann auch ein schlauchartiges Element mit einer geschlossenen Umfangswandung eingesetzt werden, wobei die Wandstärke des schlauchartigen Elements variiert, insbesondere sich in distale Richtung des Transportelements reduziert.

Bei dem erfindungsgemäßen Instrument kann ferner vorgesehen sein, dass das zylindrische Element aus einem Verbundmaterial gebildet bzw. hergestellt ist. Das Verbundmaterial kann wenigstens zwei Materialkomponenten aufweisen. Die Materialkomponenten sind vorzugsweise sichtbar voneinander getrennt. Vorteilhaft ist es, wenn eine erste Materialkomponente eine höhere Röntgensichtbarkeit oder elektrische Leitfähigkeit als eine zweite Materialkomponente aufweist. Die Verwendung eines Verbundmaterials, das elektrisch leitende Eigenschaften aufweist, ermöglicht den Einsatz des Instruments als Elektrode, beispielsweise zur elektrischen Stimulation oder zur elektrischen Gewebeablation. Eine höhere Röntgensichtbarkeit ist für alle Varianten des medizinischen Instruments, insbesondere für ein Thrombektomiedevice, einen Flow Diverter und/oder einen Blutfilter, vorteilhaft. Durch die erhöhte Röntgensichtbarkeit wird der Einsatz des Instruments erleichtert, da für den Operateur die Position des Instruments gut erkennbar ist.

Bei einer weiteren bevorzugten Ausgestaltung des erfindungsgemäßen Instruments ist die erste Materialkomponente koaxial zur und radial innerhalb der zweiten Materialkomponente angeordnet. Diese Gestaltung ergibt sich vorzugsweise aus dem Herstellungsprozess des Instruments, bei welchem das zylindrische Element aus einem einzigen Draht hergestellt wird, der aus einem Verbundmaterial aufgebaut ist. Der Draht kann einen Kerndraht aufweisen, der die erste Materialkomponente aufweist. Der Kerndraht kann als Volldraht oder als Hohldraht, d.h. mit einem Durchgangskanal, ausgebildet sein. Der Kerndraht ist mit einer Umhüllung aus der zweiten Materialkomponente versehen. Beispielsweise kann der Kerndraht Platin oder eine Platinlegierung oder Tantal oder eine Tantallegierung aufweisen. Die Verwendung von Platin ist insbesondere zur Erhöhung der elektrischen Leitfähigkeit vorteilhaft. Dabei kann die Umhüllung aus Platin oder einer Platinlegierung bestehen. Die Umhüllung kann eine Nickel-Titan-Legierung aufweisen, wobei die Umhüllung im Wesentlichen tragende Eigenschaften hat, also die mechanische Stabilität des Instruments gestaltet. Durch das Einbringen von Längsschlitzen in den Draht werden die Stege des Funktionselements freigeschnitten, die wegen der Verbundmaterialstruktur mehrschichtig ausgebildet sind. Der Kerndraht wird durch das Freischneiden der Stege zu einer inneren Schicht der Stege, während die Umhüllung eine äußere Schicht der Stege bildet.

Gemäß einem nebengeordneten Aspekt beruht die Erfindung auf dem Gedanken, ein Verfahren zur Herstellung eines medizinisches Instruments für intravaskuläre Eingriffe aus einem zylindrischen Element anzugeben, das über einen Katheter in einen Körperhohlraum einführbar ist, wobei ein distaler Endabschnitt des zylindrischen Elements durch einen materialabtragenden Arbeitsschritt strukturiert wird derart, dass ein radial expandierbarer Funktionsabschnitt des Instruments einstückig mit einem proximal angeordneten Führungsabschnitt ausgebildet wird. Gemäß einem weiteren nebengeordneten Aspekt beruht die Erfindung auf dem Gedanken, ein Verfahren zur Herstellung eines medizinischen Instruments für intravaskuläre Eingriffe aus einem zylindrischen Element anzugeben, das durch einen Katheter in einen Körperhohlraum einführbar ist, wobei ein distaler Endabschnitt des zylindrischen Elements durch einen materialabtragenden Bearbeitungsschritt strukturiert wird derart, dass aus einem Vollmaterial, insbesondere einem Volldraht, ein radial expandierbarer Funktionsabschnitt des Instruments gebildet wird.

Bei einer bevorzugten Ausführungsform des erfindungsgemäßen Verfahrens werden durch den materialabtragenden Bearbeitungsschritt zusätzlich Einschnitte in den Führungsabschnitt eingebracht, der einstückig mit dem Funktionsabschnitt ausgebildet ist. Auf diese Weise kann durch einen einzigen Bearbeitungsschritt sowohl der expandierbare Funktionsabschnitt, als auch der Führungsabschnitt gebildet werden. Dies spart Zeit bei der Produktion des medizinischen Instruments.

Bei einer weiteren bevorzugten Ausführungsform des erfindungsgemäßen Verfahrens umfasst der materialabtragende Bearbeitungsschritt Laserschneiden. Der Einsatz eines Lasers für den strukturierenden Bearbeitungsschritt ist vorteilhaft, da auf diese Weise besonders kleine Strukturen geschnitten werden können. Hinzu kommt, dass die Laserbearbeitung besonders glatte und präzise Schneidkanten zulässt, wodurch eine aufwändige Nachbearbeitung der Schnittflächen vermieden wird. Ferner ist die Materialbearbeitung durch Laserschneiden besonders schnell durchführbar, so dass die Produktionsgeschwindigkeit erhöht wird.

Die im Zusammenhang mit der eingangs erläuterten Instrument beschriebenen vorteilhaften Weiterbildungen und Vorteile gelten in analoger Weise für das erfindungsgemäße Herstellungsverfahren.

Die Erfindung wird im Folgenden anhand von Ausführungsbeispielen unter Bezugnahme auf die beigefügten, schematischen Zeichnungen näher erläutert. Darin zeigen
- Fig. 1:: eine perspektivische Ansicht des Führungsabschnittes des zylindrischen Elements, der nach einem Ausführungsbeispiel des erfindungsgemäßen Verfahrens mit Nuten zur Erhöhung der Biegeflexibilität ausgestattet ist, wobei das zylindrische Element als Volldraht ausgebildet ist;
- Fig. 2:: eine perspektivische Ansicht des Führungsabschnittes, der nach einem Ausführungsbeispiel des erfindungsgemäßen Verfahrens mit Einschnitten zur Erhöhung der Biegeflexibilität ausgestattet ist, wobei das zylindrische Element als Hohldraht ausgebildet ist;
- Fig. 3:: eine Seitenansicht des zylindrischen Elements eines erfindungsgemäßen medizinischen Instruments, nach einem bevorzugten Ausführungsbeispiel, wobei der Funktionsabschnitt eine greiferartige Struktur aufweist;
- Fig. 4:: eine Seitenansicht eines erfindungsgemäßen medizinischen Instruments, nach einem weiteren bevorzugten Ausführungsbeispiel, wobei der Funktionsabschnitt eine netzartige Struktur aufweist;
- Fig. 5:: eine Seitenansicht des Instruments gemäß Fig. 4, wobei ein distaler Endabschnitt im Detail dargestellt ist;
- Fig. 6:: eine perspektivische Ansicht des distalen Endabschnitts gemäß Fig. 5;
- Fig. 7:: eine Vorderansicht des distalen Endabschnittsgemäß Fig. 5;
- Fig. 8a, 8b:: eine Detailansicht des Führungsabschnittes des zylindrischen Elements nach einem bevorzugten Ausführungsbeispiel, der Bereiche mit unterschiedlichen Vorzugsrichtungen für die Biegeflexibilität aufweist;
- Fig. 9a: eine Seitenansicht eines medizinischen Instruments nach einem weiteren bevorzugten Ausführungsbeispiel, wobei der Führungsabschnitte des zylindrischen Elements Einschnitte zur Erhöhung der Biegeflexiblität aufweist;
- Fig. 9b: eine Detailansicht des medizinischen Instruments gemäß Fig. 9a;
- Fig. 10: eine Seitenansicht des zylindrischen Elements des medizinischen Instruments gemäß Fig. 9a;
- Fig. 11: eine perspektivische Ansicht des zylindrischen Elements gemäß Fig. 10;
- Fig. 12a: eine Seitenansicht eines medizinischen Instruments nach einem weiteren bevorzugten Ausführungsbeispiel, das einen Schlauch als Flexionselement umfasst;
- Fig. 12b: eine Detailansicht des medizinischen Instruments gemäß Fig. 12a;
- Fig. 13a: eine Seitenansicht eines medizinischen Instruments nach einem weiteren bevorzugten Ausführungsbeispiel, das einen Coil als Flexionselement aufweist;
- Fig. 13b: eine Detailansicht des Instruments gemäß Fig. 13a;
- Fig. 14: eine Querschnittsansicht eines Funktionsabschnitts der Erfindung gemäß einem bevorzugten Ausführungsbeispiel;
- Fig. 15: eine Querschnittsansicht eines Stegs des Funktionsabschnitts gemäß Fig. 14;
- Fig. 16: eine Draufsicht auf einen Teil der Umfangswandung gemäß Fig. 15;
- Fig. 17a-c: Schnittdarstellungen der Umfangswandung gemäß Fig. 16; und
- Fig. 18: eine Röntgendarstellung der Umfangswandung gemäß Fig. 16.

Mit dem erfindungsgemäßen Verfahren sind medizinische Instrumente für intravaskuläre Eingriffe, insbesondere Mikrodrahtretriever, Körbe, Filter oder Greifer herstellbar, die zur Entfernung von Blutgerinnseln aus Blutgefäßen oder zum Halten und Führen von Implantaten, beispielsweise Stents, eingesetzt werden. Es werden sowohl das Herstellungsverfahren im Zusammenhang mit den vorstehend genannten Instrumenten als auch die vorstehend genannten Instrumente als solche offenbart und beansprucht. Um die Fertigungskosten zu reduzieren und gleichzeitig zu vermeiden, dass Teile des Instruments versagen, ist im Rahmen der Erfindung vorgesehen, ein einziges Ausgangsmaterial zur Bildung des Instruments einzusetzen.

Das Instrument 10 weist im Allgemeinen ein zylindrisches Element 11, insbesondere ein Drahtelement, auf. Das Instrument 10 kann vollständig durch das zylindrische Element 11 gebildet sein. Das zylindrische Element 11 ist geeignet, um über einen Katheter in ein Körperhohlorgan eingeführt zu werden. Das zylindrische Element 11 weist einen Funktionsabschnitt 13 auf, der durch einen distalen Endabschnitt des zylindrischen Elements 11 gebildet ist. Der distale Endabschnitt, der auch ein distales Ende 22 des medizinischen Instruments 10 bildet, ist strukturiert, so dass sich der Funktionsabschnitt 13 bildet. Der Funktionsabschnitt 13 ist radial expandierbar. Mit anderen Worten ist der distale Endabschnitt 12 des zylindrischen Elements 11 derart strukturiert, insbesondere derart materialabtragend bearbeitet, dass ein radial expandierbarer Funktionsabschnitt gebildet ist. Der Funktionsabschnitt ist einstückig mit einem Führungsabschnitt 14 des zylindrischen Elements 11 ausgebildet. Das zylindrische Element 11 ist also einteilig ausgebildet und umfasst einen radial expandierbaren Funktionsabschnitt 13 und einen durchmesserstabilen Führungsabschnitt 14. Der Führungsabschnitt 14 schließt sich proximal an den Funktionsabschnitt 13 an. Aufgrund der einstückigen Verbindung bildet der Funktionsabschnitt 13 eine stoffschlüssige Verlängerung des Führungsabschnitts 14. Das Material des Führungsabschnitts 14 geht kontinuierlich in das Material des Funktionsabschnitts 13 über. Im Bereich des Übergangs ist das Materialgefüge unverändert. Dies unterscheidet den Übergang bspw. von einer Schweißverbindung.

Der durchmesserstabile Führungsabschnitt 14 weist im Wesentlichen einen konstanten Querschnittsdurchmesser auf. Damit stabilisiert der Führungsabschnitt 14 den expandierbaren Funktionsabschnitt 13. Da der Funktionsabschnitt 13 und der Führungsabschnitt 14 einstückig ausgebildet sind, ist die Herstellung vereinfacht. Eine aufwändige Verbindung zwischen dem Funktionselement 13 und dem Führungsabschnitt 14 entfällt. Das erfindungsgemäße kann leicht durch einen Katheter geschoben werden, da wegen der einstückigen Verbindung keine Schweißoder Lötstellen vorliegen, die über den Außenumfang des zylindrischen Elements vorstehen und zu einer erhöhten Reibung an der Innenumfangswand eines Katheters führen.

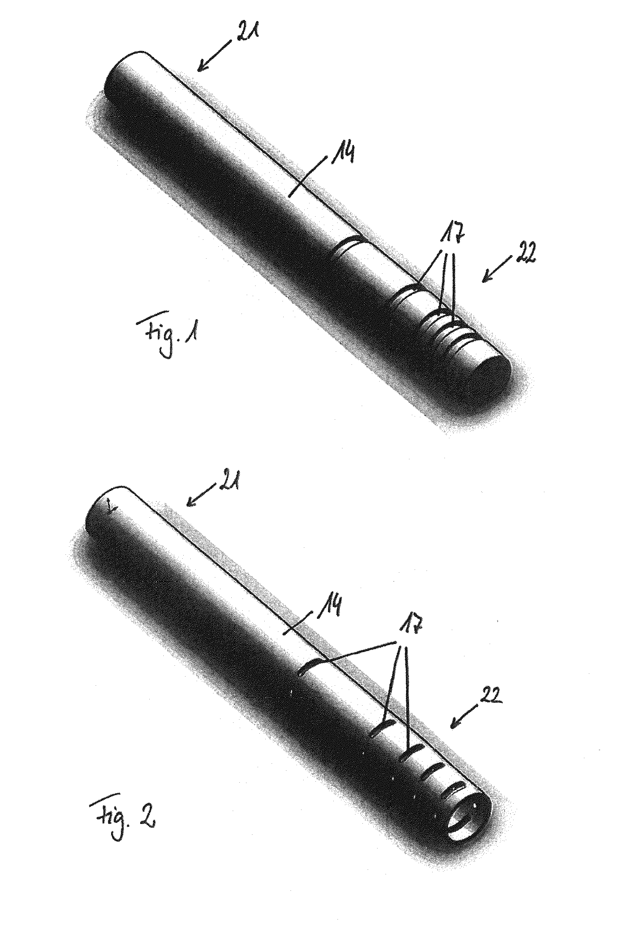

Fig. 1, 2 zeigen den Führungsabschnitt 14 ohne den Funktionsabschnitt 13, um den Querschnitt des Führungsabschnitts 14 (voll/hohl) darzustellen.

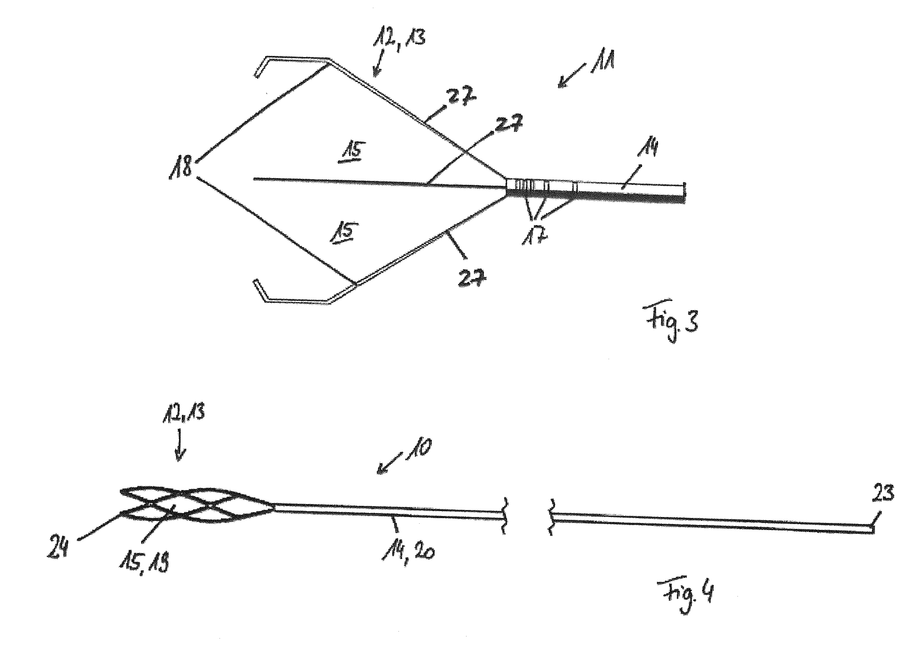

Fig. 3 zeigt in einer Seitenansicht ein zylindrisches Element 11 eines erfindungsgemäßen medizinischen Instruments 10 nach einem bevorzugten Ausführungsbeispiel. Gut erkennbar ist der Führungsabschnitt 14 und der distal an den Führungsabschnitt 14 anschließende Funktionsabschnitt 13, der einen distalen Endabschnitt 12 des zylindrischen Elements 11 bildet. Der Funktionsabschnitt 13 weist eine greiferartige Struktur mit Greiffingern bzw. Greifarmen 18 auf, wobei Fig. 3 den Funktionsabschnitt 13 im expandierten Zustand zeigt. Der Funktionsabschnitt 13 ist dadurch gebildet, dass in das zylindrische Element 11 entlang des distalen Endabschnitts 12 Längsschnitte 15 eingebracht sind, die das Material des distalen Endabschnitts 12 durchtrennen. Die Längsschnitte 15 erstrecken sich über die gesamte Länge des distalen Endabschnitts 12. Die Greifarme sind als einzelne Stege 27 ausgebildet.

Allgemein sind die proximalen Enden der Stege 27 jeweils mit dem Führungsabschnitt 14 verbunden und bilden eine einstückige Verlängerung des Führungsabschnittes 14. Die Stege 27 fluchten im komprimierten Zustand mit der zylindrichen Außenkontur des Führungsabschnitts 14.

Bei dem Beispiel gemäß Fig. 3 sind die distalen Enden der Stege 27 jeweils freie Enden. Die freien Enden sind profiliert und bilden Greifhaken, mit denen bspw. ein Implantat gefasst werden kann.

Alternativ kann vorgesehen sein, dass entlang des distalen Endabschnitts 12 mehrere Längsschnitte 15 versetzt zueinander angeordnet sind, so dass sich eine im Wesentlichen netzartige Struktur des Funktionsabschnitts 13 ergibt. Ein derartiges Ausführungsbeispiel ist in Fig. 4 gezeigt. Es ist erkennbar, dass der Funktionsabschnitt 13 im Wesentlichen stentähnlich ausgebildet ist, wobei die Längsschnitte 15 im expandierten Zustand des Funktionsabschnitts 13 Zellen 19 der stentartigen Struktur bilden. Das medizinische Instrument gemäß Fig. 4 ist einstückig ausgebildet. Insbesondere weist das medizinische Instrument 10 ein einziges zylindrisches Element 11 auf, das in einem distalen Endabschnitt 12 den Funktionsabschnitt 13 umfasst. Der Funktionsabschnitt 13 ist im Wesentlichen an einem distalen Ende 24 des medizinischen Instruments 10 ausgebildet. In proximaler Richtung schließt sich an den Funktionsabschnitt 13 der Führungsabschnitt 14 an, der bei dem Ausführungsbeispiel gemäß Fig.4 gleichzeitig ein Transportelement 20, insbesondere einen Transportdraht, bildet. Der Führungsabschnitt 14 bzw. das Transportelement 20 sind durchmesserstabil, also nicht expandierbar.

Die stentartige bzw. röhrchenartige Gitterstruktur ist aus mehreren Stegen 27 gebildet, die sich zur Bildung von Zellen kreuzen. Die Gitterstruktur ist in an sich bekannter Weise komprimierbar und expandierbar und bildet ein Rekanalisationselement. Die Stege 27 gehen kontinuierlich in den Führungsabschnitt 14 über und bilden eine einstückige Verlängerung des Führungsabschnitts 14. Die Gitterstruktur und der Führungsabschnitt 14 sind also einstückig ausgebildet.

Konkret bilden die Stege, die direkt an den Führungsabschnitt 14 angrenzen, Übergangsstege 27b, die parallel zur Längsachse des Funktionselementes 13 angeordnet sind. Vorliegend sind 4 Übergangsstege 27b gezeigt. Es ist möglich, einen einzigen oder mehr als einen Übergangssteg 27b, insbesondere mehr als 2, mehr als 3, mehr als 4, mehr als 5, mehr als 6, mehr als 7 oder mehr als 8 Übergangsstege 27b auszubilden. Die Übergangsstege 27b sind mit Gitterstegen 27c verbunden, die schräg zur Längsachse des Funktionselementes 13 angeordnet sind und sich zur Bildung von Gitterzellen kreuzen.

Um die Manövrierfähigkeit des medizinischen Instruments 10 zu verbessern, kann vorgesehen sein, dass das zylindrische Element 11 im Führungsabschnitt 14 Einschnitte 17 aufweist, die die Biegeflexibilität der Verlängerung 14 verändern. Bei dem greifartigen Ausführungsbeispiel gemäß Fig. 3 sind beispielsweise in der Verlängerung 14 mehrere Einschnitte 17 angeordnet, die in einem distalen Bereich, also einem dem Funktionsabschnitt 13 näheren Bereich, der Verlängerung 14 die Biegeflexibilität erhöhen.

Die Erhöhung der Biegeflexibilität beruht auf dem Prinzip, entlang der durchmesserstabilen Verlängerung 14 des zylindrischen Elements 11 Materialschwächungen bzw. Materialdurchbrüche vorzusehen, so dass der Querschnittsdurchmesser der Verlängerung 14 bereichsweise reduziert ist. Dieses Prinzip wird im Folgenden anhand der Fig. 1 und 2 erläutert.

Die Fig. 1 und 2 zeigen jeweils unterschiedliche Ausführungsbeispiele des Führungsabschnitts 14, wobei der Führungsabschnitt 14 ohne den Funktionsabschnitt 13 dargestellt ist. Dies dient der Veranschaulichung. Grundsätzlich ist vorgesehen, dass der Führungsabschnitt 14 einteilig mit dem Funktionsabschnitt 13 verbunden ist.

Der Führungsabschnitt 14 ist bei dem Ausführungsbeispiel gemäß Fig. 1 als Volldraht ausgebildet. In einem distalen Bereich 22 des Führungsabschnitts 14 sind mehrere umlaufende Einschnitte 17 vorgesehen, die im Wesentlichen Ringnuten bilden. Es ist erkennbar, dass entlang des Führungsabschnitts 14 mehrere Einschnitte 17 angeordnet sind, wobei sich der Abstand zwischen den Einschnitten 17 in Richtung zu einem proximalen Bereich 21 der Verlängerung 14 erhöht. Mit anderen Worten reduziert sich der Abstand zwischen benachbarten Einschnitten 17 in Richtung des distalen Bereichs 22 bzw. in Richtung des Funktionselements 13. So wird erreicht, dass sich die Biegeflexibilität des zylindrischen Elements, insbesondere des Führungsabschnitts 14, von proximal nach distal erhöht. Die relativ größere Biegeflexibilität im distalen Bereich 22 ist vorteilhaft, um die Manövrierfähigkeit des medizinischen Instruments 10 zu verbessern. Im proximalen Bereich 21 ist eine vergleichsweise niedrige Biegeflexibilität, d.h. eine hohe Biegesteifigkeit, vorteilhaft, um axiale Kräfte auf den Funktionsabschnitt 13 übertragen zu können.

Das Ausführungsbeispiel gemäß Fig. 2 entspricht in wesentlichen Teilen dem Ausführungsbeispiel gemäß Fig. 1, wobei jedoch der Führungsabschnitt 14 durch einen Hohldraht gebildet ist. Die Einschnitte 17 sind im Unterschied zu dem Ausführungsbeispiel gemäß Fig. 1 nicht als Materialschwächungen, insbesondere Ringnuten, sondern als Materialdurchbrüche ausgebildet. Insbesondere sind jeweils mehrere in Umfangsrichtung fluchtende Schlitze vorgesehen. Jeweils in Umfangsrichtung zueinander fluchtende Schlitze bilden einen Schlitzkreis, wobei mehrere Schlitzkreise in axialer Richtung der Verlängerung 14 beabstandet zueinander angeordnet sind. Die Schlitzkreise entsprechen im Wesentlichen den Ringnuten gemäß Fig. 1. So ist analog vorgesehen, dass die Schlitzkreise zueinander unterschiedliche Abstände aufweisen, insbesondere sich der Abstand der unmittelbar benachbarten Schlitzkreise in Richtung des proximalen Bereichs 21 erhöht.

In diesem Zusammenhang wird darauf hingewiesen, dass der Führungsabschnitt 14, wie beispielsweise in Fig. 1 und 2 dargestellt, ein Transportelement 20 bilden kann. Der proximale Bereich 21 kann also am proximalen Ende des medizinischen Instruments 10 angeordnet sein bzw. das proximale Ende 23 bilden.

In den Fig. 1 und 2 sind die Einschnitte 17 ringförmig oder schlitzartig in Umfangsrichtung des Führungsabschnitts 14 bzw. des zylindrischen Elements 11 angeordnet. Andere Formen der Einschnitte 17 sind möglich. Beispielsweise können die Einschnitte 17 helixförmig bzw. spiralförmig um die Längsachse des Führungsabschnitts 14 angeordnet sein. Zur Einstellung der Biegeflexibilität kann nicht nur der Abstand zwischen den einzelnen Einschnitten 17 variiert werden. Es ist auch möglich, die Breite und/oder Tiefe der Einschnitte 17 zu verändern, um eine gewünschte Biegeflexibilität einzustellen. Die Breite der Einschnitte 17 wird vorzugsweise in axialer Richtung, d.h. parallel zur Längsachse, des Führungsabschnitts 14 ermittelt. Die Tiefe der Einschnitte 17 ergibt sich entlang des Radius des Führungsabschnitts 14.

Einzelne Einschnitte 17 können eine Breite aufweisen, die höchstens um das 10-fache, insbesondere höchstens um das 8-fache, insbesondere höchstens um das 6-fache, insbesondere höchstens um das 4-fache, insbesondere höchstens um das 2-fache, größer als die Tiefe des jeweiligen Einschnitts 17 ist. Umgekehrt kann die Tiefe eines einzelnen Einschnitts 17 auch um höchstens das 10-fache, insbesondere höchstens das 8-fache, insbesondere höchstens das 6-fache, insbesondere höchstens das 4-fache, insbesondere höchstens das 2-fache, der Breite des jeweiligen Einschnitts 17 entsprechen. Ferner können Breite und Tiefe eines Einschnitts 17 identisch sein.

Das Verhältnis zwischen dem Abstand d zwischen zwei benachbarten Einschnitten 17 und der Breite b an einer der beiden Einschnitte 17 kann höchstens 2:1 und/oder wenigstens 1:1, insbesondere wenigstens 1:2, insbesondere wenigstens 1:5, insbesondere wenigstens 1:10, insbesondere wenigstens 1:20, insbesondere wenigstens 1:50, insbesondere wenigstens 1:70, insbesondere wenigstens 1:90, insbesondere wenigstens 1:100, betragen.

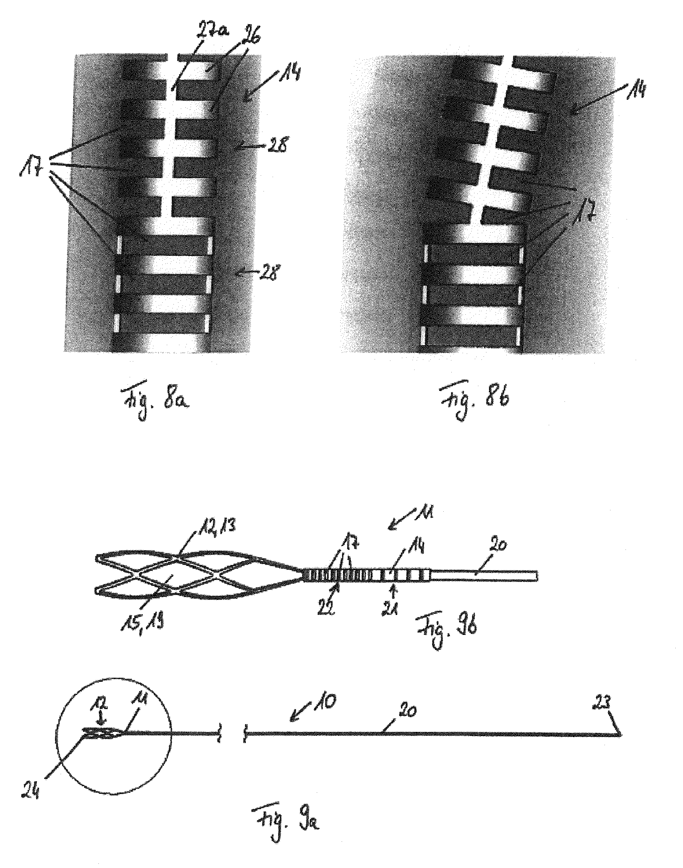

In den Fig. 8a und 8b ist ein weiteres Ausführungsbeispiel für die Gestaltung eines Führungsabschnitts 14 gezeigt, der mehrere Einschnitte 17 aufweist. Der Führungsabschnitt 14 ist als Hohldraht ausgebildet. Die Einschnitte 17 bilden Materialdurchbrüche. Konkret erstrecken sich die Einschnitte 17 derart durch den Hohldraht, dass in Längsrichtung des Führungsabschnitts 14 benachbart und beabstandet voneinander angeordnete Ringe 26 gebildet sind, die durch zwei parallele, längsaxial ausgerichtete Stegelemente 27a miteinander verbunden sind. Der Führungsabschnitt 14 weist mehrere, insbesondere zwei Blöcke 28 von Einschnitten 17 auf, wobei die Einschnitte 17 in den beiden Blöcken 28 jeweils unterschiedlich orientiert sind. Konkret sind die Einschnitte 17 in einem Block 28 gegenüber den Einschnitten 17 des anderen Blocks 28 um die Längsachse des Führungsabschnitts 14 verdreht. Insbesondere sind die Einschnitte 17 blockweise um 90 Grad verdreht. So kann die Biegeflexibilität des Führungsabschnitts 14 in besonderen Vorzugsrichtungen eingestellt werden. Fig. 8b zeigt deutlich, dass durch die Verdrehung der Einschnitte 17 zueinander der Führungsabschnitt 14 blockweise in eine erste Richtung eine höhere Biegeflexibilität aufweist, als in eine zweite Vorzugsrichtung. Die erste Vorzugsrichtung ist in der Zeichnung gemäß Fig. 8b in der Zeichenebene angeordnet. Die zweite Vorzugsrichtung erstreckt sich in der Zeichnung gemäß Fig. 8b senkrecht zur Zeichenebene.

Grundsätzlich können über den Umfang des Führungsabschnitts 14 verteilt mehrere Einschnitte 17 vorgesehen sein. Vorzugsweise sind jeweils wenigstens 2, insbesondere wenigstens 3, insbesondere wenigstens 4, insbesondere wenigstens 5 Einschnitte über den Umfang der Verlängerung 14 verteilt angeordnet. Die vorgenannten Zahlen beziehen sich auf einen einzelnen Ring aus Einschnitten 17 und berücksichtigen nicht axial zueinander beabstandete Einschnitte 17.

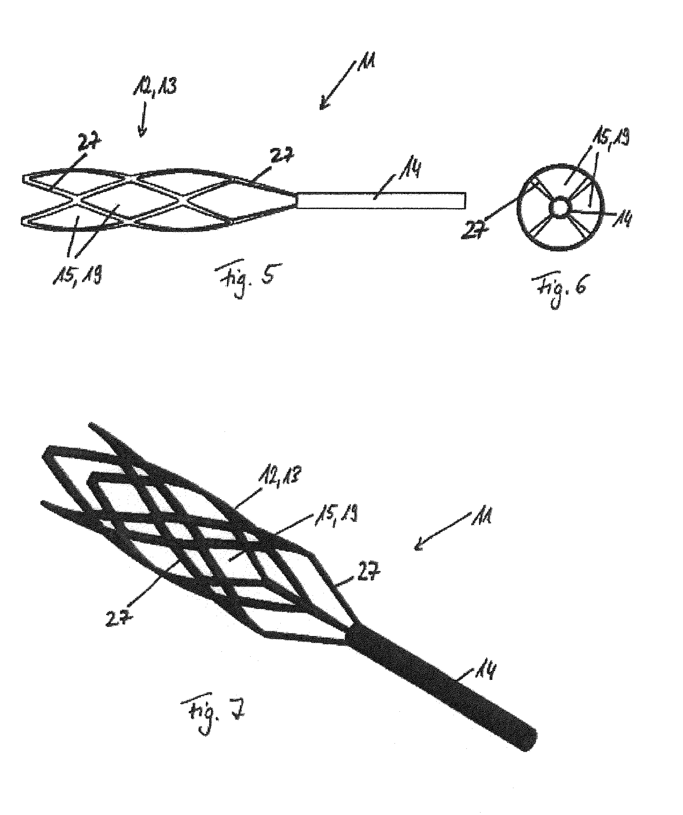

Fig. 5 zeigt eine Seitenansicht eines zylindrischen Instruments 11 gemäß Fig. 4, wobei die netzartige Struktur des Funktionsabschnitts 13 gut erkennbar ist. An den Funktionsabschnitt 13 schließt sich unmittelbar der Führungsabschnitt 14 an, der bei dem Ausführungsbeispiel gemäß Figuren 4-7 unstrukturiert ist, d.h. keine Einschnitte 17 aufweist. Es kann vorgesehen sein, dass der Führungsabschnitt 14 in einem distalen Bereich, der sich direkt an den Funktionsabschnitt 13 anschließt, eine Strukturierung, insbesondere eine Oberflächenstrukturierung aufweist, wodurch die Biegeflexibilität des Führungsabschnitts 14, insbesondere des distalen Bereichs des Führungsabschnitts 14, erhöht wird. Die Oberflächenstrukturierung kann durch das Einbringen von Einschnitten 17 realisiert werden, die sich zumindest teilweise in Umfangsrichtung des zylindrischen Elements 11 erstrecken.

In den Figuren 6 und 7, die weitere Ansichten des zylindrischen Elements 11 gemäß Fig. 5 zeigen, ist gut erkennbar, dass das zylindrische Element 11 als Hohldraht ausgebildet ist, der im distalen Endabschnitt 12 derart strukturiert ist, dass der radial expandierbare Funktionsabschnitt 13 gebildet ist.

In den Fig. 9a und 9b ist ein weiteres Ausführungsbeispiel eines medizinischen Instruments 10 dargestellt, wobei Fig. 9b eine Detailansicht des Instruments 10 gemäß Fig. 9a zeigt. Das dargestellte Instrument 10 weist ein zylindrisches Element 11 auf, das am distalen Ende 24 des Instruments 10 angeordnet ist. Das zylindrische Element 11 ist proximal mit einem Transportelement 20 gekoppelt, insbesondere fest mit dem Transportelement 20 verbunden. Das Transportelement 20, das insbesondere als Transportdraht ausgebildet sein kann, erstreckt sich vom zylindrischen Element 11 bis zum proximalen Ende des medizinischen Instruments 10. Mit anderen Worten besteht das medizinische Instrument 10 aus dem Transportelement 20 und dem zylindrischen Element 11.

Das zylindrische Element 11 weist einen Endabschnitt 13 auf, der derart strukturiert ist, dass ein stentartiger Funktionsabschnitt 12 gebildet ist. Im Hinblick auf die konstruktive Gestaltung des Funktionsabschnitts 12 wird auf die Beschreibung vorhergehender Ausführungsbeispiel verwiesen. An den Funktionsabschnitt 12 schließt sich unmittelbar der Führungsabschnitt 14 an. Der Führungsabschnitt 14 bildet ein Transportdrahtsegment, das mit einem gesonderten Transportdraht verbunden ist. Konkret ist das Transportdrahtsegment als Hohldraht gebildet, wobei der Transportdraht in den Hohldraht eingeschoben ist. Der Transportdraht weist also einen Querschnittsdurchmesser auf, der dem Innendurchmesser des Hohldrahts bzw. des Führungsabschnitts 14 entspricht. Es bilden also der Funktionsabschnitt 13 und das Transportdrahtsegment eine Einheit, die mit dem Transportdraht als gesondertes Bauteil verbunden ist. Im Unterschied dazu bilden der Funktionsabschnitt 13 und der Transportdraht der vorangegangenen Beispiele eine Einheit, wobei der gesamte Transportdraht dem Führungsabschnitt 14 entspricht.

Entlang des Führungsabschnitts 14 sind mehrere Einschnitte angeordnet, wobei die Dichte von benachbarten Einschnitten 17 in einem distalen Bereich 22 des Führungsabschnitts 14 höher als in einem proximalen Bereich 21 ist. Mit anderen Worten sind die Einschnitte 17 im distalen Bereich 22 näher aneinander angeordnet, als im proximalen Bereich 21. Dies bezieht sich auf den Abstand der Einschnitte 17 in längsaxialer Richtung zueinander.

Im Allgemeinen gilt für alle Ausführungsbeispiele, dass das zylindrische Element 11 zwei Abschnitte aufweist, die unmittelbar ineinander übergehen können. Ein distaler Endabschnitt 13 bildet das expandierbare Funktionselement 12. Ein proximaler Endabschnitt bildet den Führungsabschnitt 14. Der Führungsabschnitt 14 ist durchmesserstabil, also nicht-expandierbar. Das Verhältnis zwischen dem Querschnittsdurchmesser des Führungsabschnitts 14 und dem Querschnittsdurchmesser des voll expandierten Funktionsabschnitts 13 beträgt vorzugsweise höchstens 1:2, insbesondere höchstens 1:3; insbesondere höchstens 1:4, insbesondere höchstens 1:5, insbesondere höchstens 1:10, insbesondere höchstens 1:15, insbesondere höchstens 1:18, insbesondere höchstens 1:20. Der Führungsabschnitt 14 kann dabei einen Querschnittsdurchmesser im Bereich von 0,2 mm bis 0,6 mm, insbesondere im Bereich von 0,3 mm bis 0,5 mm, vorzugsweise 0,4 mm, aufweisen. Entsprechend kann vorgesehen sein, dass der Funktionsabschnitt 13 im voll expandierten Zustand, d.h. im Ruhezustand, einen Querschnittsdurchmesser im Bereich von 2 mm bis 8 mm, insbesondere im Bereich von 3 mm bis 7 mm, insbesondere im Bereich von 3,5 mm bis 6 mm, aufweist.

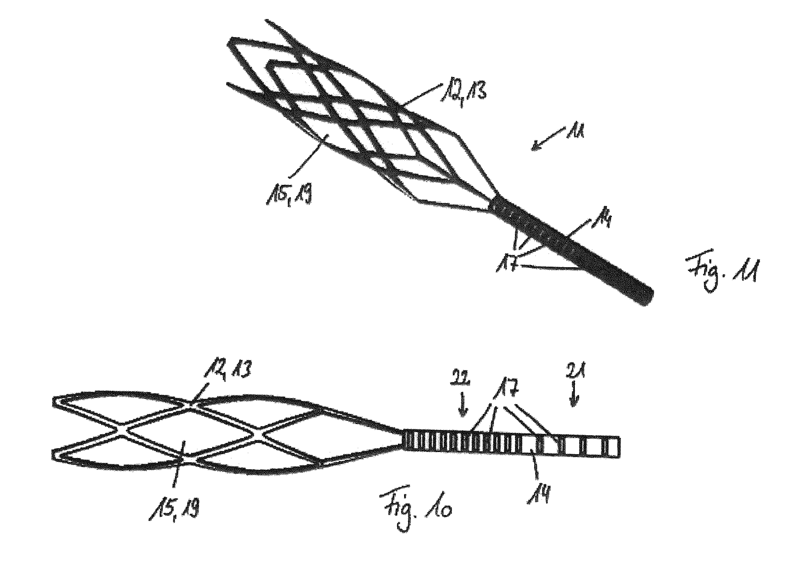

Die Fig. 10 und 11 zeigen das zylindrische Element 11 des medizinischen Instruments 10 gemäß Fig. 9a und 9b im Detail. Dabei ist das zylindrische Element 11 in Fig. 10 in einer Seitenansicht und in Fig. 11 in einer perspektivischen Ansicht dargestellt.

Hinsichtlich der Längenverhältnisse des zylindrischen Elements 11 ist vorgesehen, dass der Führungsabschnitt 14 vorzugsweise eine axiale Länge aufweist, die wenigstens dem 2-fachen, insbesondere wenigstens dem 3-fachen, insbesondere wenigstens dem 5-fachen, insbesondere wenigstens dem 8-fachen, insbesondere wenigstens dem 10-fachen, insbesondere wenigstens dem 15-fachen, insbesondere wenigstens dem 20-fachen, der Länge des Funktionsabschnitts 13 entspricht. Beispielsweise kann der Funktionsabschnitt 13 eine Länge von wenigstens 10 mm, insbesondere wenigstens 20 mm, insbesondere wenigstens 40 mm und/oder höchstens 100 mm, insbesondere höchstens 70 mm, insbesondere höchstens 60 mm, insbesondere höchstens 50 mm, insbesondere höchstens 30 mm, aufweisen. Der Führungsabschnitt 14 kann eine Länge von wenigstens 50 mm, insbesondere wenigstens 100 mm, insbesondere wenigstens 200 mm, insbesondere wenigstens 300 mm, insbesondere wenigstens 400 mm, und/oder höchstens 800 mm, insbesondere höchstens 600 mm, insbesondere höchstens 500 mm, insbesondere höchstens 300 mm, insbesondere höchstens 200 mm, aufweisen. Die vorgenannten Längenangaben für den Führungsabschnitt 14 gelten für die zweiteilige Variante des medizinischen Instruments, bei der der Führungsabschnitt 14 mit dem Transportdraht verbunden ist. Wenn, wie es eine bevorzugte Ausführungsform vorsieht, der Führungsabschnitt 14 gleichzeitig den Transportdraht bildet, der Transportdraht also mit dem Führungsabschnitt 14 identisch ist, kann der Führungsabschnitt 14 bzw. das Transportelement 20 eine Länge aufweisen, die wenigstens 800 mm, insbesondere wenigstens 1000 mm, insbesondere wenigstens 1500 mm, insbesondere wenigstens 2000 mm, beträgt. Als Obergrenze für die Länge des Führungsabschnitt 14 bzw. des Transportelements 20 sind höchstens 3000 mm, insbesondere höchstens 2500 mm, insbesondere höchstens 2000 mm, insbesondere höchstens 1500 mm, vorgesehen.

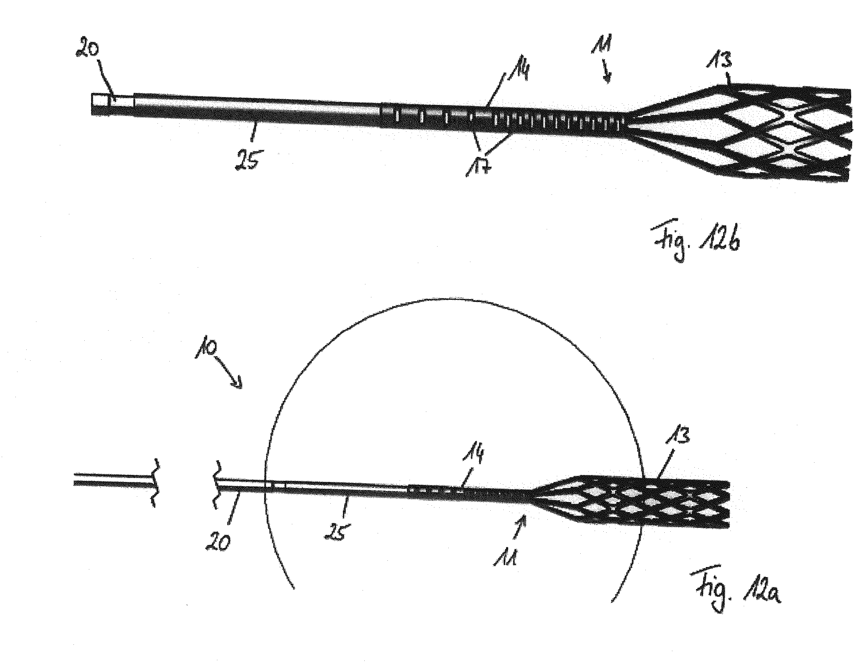

In den Fig. 12a und 12b ist eine zweiteilige Variante des medizinischen Instruments 10 dargestellt. Das medizinische Instrument 10 umfasst ein zylindrisches Element 11 und ein Transportelement 20, wobei das Transportelement 20 mit dem zylindrischen Element 11, insbesondere dem Führungsabschnitt 14 des zylindrischen Elements 11 fest verbunden ist. Im Allgemeinen ist das zylindrische Element 11 analog zu dem Ausführungsbeispiel gemäß den Fig. 10 und 11 ausgebildet. So weist das zylindrische Element 11 einen Funktionsabschnitt 13 und einen Führungsabschnitt 14 auf, wobei der Funktionsabschnitt 13 radial expandierbar ist. Der Führungsabschnitt 14 ist durchmesserstabil und weist mehrere in Längsrichtung beabstandet zueinander angeordnete Einschnitte 17 auf, die die Biegeflexibilität der Verlängerung 14 beeinflussen. Die Verbindung zwischen dem zylindrischen Element 11 bzw. dem Führungsabschnitt 14 und dem Transportelement 20 erfolgt vorzugsweise durch Kleben, Schweißen und/oder eine mechanische Arretierung. Beispielsweise kann eine formschlüssige Verbindung zwischen dem Transportelement 20 und dem zylindrischen Element 11 vorgesehen sein. Das zylindrische Element 11 kann im Führungsabschnitt 14 einen Hinterschnitt aufweisen, in den ein Vorsprung des Transportelements 20 eingreift. Ferner kann die Verbindung zwischen zylindrischem Element 11 und Transportelement 20 auch durch Crimpen, Quetschen oder Einpressen erfolgen.

Bei dem Ausführungsbeispiel gemäß Fig. 12a und 12b, wobei 12b eine Detailansicht des medizinischen Instruments 10 gemäß Fig. 12a zeigt, ist das Transportelement 20 zusätzlich mit einem Flexionselement 25 ausgestattet. Das Flexionselement 25 bildet ein Mittel zur Beeinflussung der Biegeflexibilität des Transportelements 20. Im dargestellten Ausführungsbeispiel ist das Flexionselement 25 als Schlauch, insbesondere Kunststoffschlauch, ausgebildet. Das Flexionselement 25 schließt sich unmittelbar an den Führungsabschnitt 14 an. Durch das Flexionselement 25 wird im Bereich proximal des Führungsabschnitts 14 die Biegeflexibilität des Transportelements 20 verändert, vorzugsweise erhöht. Es kann auch vorgesehen sein, dass entlang des Flexionselements 25 die Biegeflexibilität kontinuierlich oder schrittweise variiert. Dies ist konstruktiv dadurch erreichbar, dass der Schlauch, der das Flexionselement 25 bildet, in längsaxialer Richtung eine kontinuierlich oder schrittweise ansteigende Wandstärke aufweist. Insbesondere kann sich die Wandstärke in Richtung distal, d.h. in Richtung des Funktionsabschnitts 13, reduzieren. So wird erreicht, dass sich die Biegeflexibilität in distale Richtung des Transportelements 20 erhöht bzw. das Transportelement 20 in proximale Richtung ausgehend vom Führungsabschnitt 14 versteift wird.

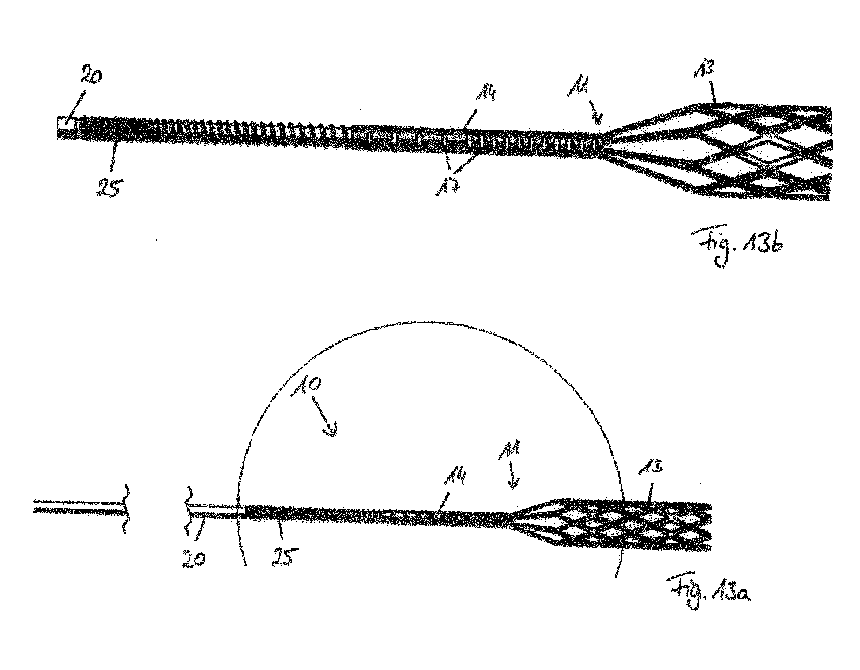

Die Veränderung der Biegeflexibilität des Transportelements 20 kann nicht nur durch ein Flexionselement 25 erreicht werden, das als Schlauch ausgebildet ist. Andere Flexionselemente 25 sind denkbar. So zeigt das Ausführungsbeispiel gemäß Fig. 13a und 13b ein im Wesentlichen identisches medizinisches Instrument 10, das sich von dem Ausführungsbeispiel gemäß Fig. 12a und 12b lediglich darin unterscheidet, dass das Flexionselement 25 als Coil ausgebildet ist. Der Coil weist ein helixförmig um die Längsachse des Transportelements 20 gewundenes Drahtelement auf, wobei die Steigung des Drahtelements bzw. der einzelnen Windungen des Drahtelements in distale Richtung ansteigt. Mit anderen Worten erhöht sich der Abstand zwischen einzelnen Windungen des Drahtelements in distale Richtung. So wird erreicht, dass an einem vom Führungsabschnitt 14 beabstandeten Ende des Flexionselements eine relativ höhere Biegesteifigkeit vorliegt als in einem Bereich des Transportelements 20, der unmittelbar an den Führungsabschnitt 14 angrenzt. Mit anderen Worten erhöht sich die Biegesteifigkeit in proximale Richtung ausgehend vom Führungsabschnitt 14 aufgrund der enger zusammenrückenden Windungen des Coildrahts. Der Coil kann im Wesentlichen als Spiralfeder ausgebildet sein. Im Allgemeinen kann der Coil federartige Eigenschaften aufweisen.

Gemeinsam mit den Einschnitten 17 im Führungsabschnitt 14 wird durch das Flexionselement 25 am Transportelement 20 erreicht, dass die Biegeflexibilität von proximal nach distal kontinuierlich ansteigt. Damit wird ein homogenes Verhalten des medizinischen Instruments 10 erreicht, insbesondere ein Knicken des Instruments 10 vermieden.

Bei der Ausführung des Flexionselements 25 als Coil kann die Änderung der Biegeflexibilität bzw. Biegesteifigkeit auch durch eine Änderung des Drahtdurchmessers des Coildrahts erreicht werden. Überdies sind Varianten denkbar, bei denen das Flexionselement 25 aus einer Kombination aus Schläuchen, Federn, Hülsen oder ähnlichen die Biegesteifigkeit bzw. Biegeflexibilität beeinflussenden Elementen gebildet ist. Bei Verwendung. Bei Verwendung von Schläuchen, auch in Kombination mit anderen Flexionselementen 25, können auch Schrumpfschläuche eingesetzt werden.

Bei den mit dem erfindungsgemäßen Verfahren hergestellten Instrumenten 10 ergeben sich folgende Vorteile:
Das zylindrische Element 11, das üblicherweise eine relativ große Gesamtlänge aufweist, ist im unbearbeiteten Zustand relativ steif. Diese Steifigkeit wird bei dem Instrument 10 zumindest entlang des Transportelements 20 beibehalten. Entlang des Führungsabschnitts 14 kann hingegen die Biegeflexibilität erhöht werden, indem Einschnitte 17 in das zylindrische Element 11 eingebracht werden.

Durch die laserschneidende Bearbeitung des distalen Endabschnitts 12 des zylindrischen Elements 11 wird der Funktionsabschnitt 13 ausgeformt. Dabei wird der Endabschnitt 12 derart strukturiert, dass der ausgeformte Funktionsabschnitt 13 radial expandierbar ist. Der Bearbeitungsschritt, der zur Bildung des Funktionsabschnitts 13 führt, und der Bearbeitungsschritt, der zur Erhöhung der Biegeflexibilität im distalen Bereich des Führungsabschnitts 14 führt, können in unterschiedlicher zeitlicher Reihenfolge oder gleichzeitig durchgeführt werden. Beispielsweise kann zuerst der Funktionsabschnitt 13 gebildet und anschließend der Führungsabschnitt 14 mit Einschnitten 17 versehen werden. Umgekehrt können die Einschnitte 17 zuerst im Führungsabschnitt 14 eingebracht werden und anschließend der Funktionsabschnitt 13 gebildet werden. Da das Transportelement 20 bzw. - bei einstückiger Ausbildung - der proximale Bereich des Führungsabschnitts 14 keine Strukturierung erfährt, weist das zylindrische Element 11 insgesamt vergleichsweise gute Gleiteigenschaften im Zuführsystem, insbesondere in einem Katheter, auf. Die guten Gleiteigenschaften werden begünstigt, wenn das gesamte Instrument 10 einstückig hergestellt ist, also keine Materialverdickungen, beispielsweise durch Schweißnähte, vorliegen.

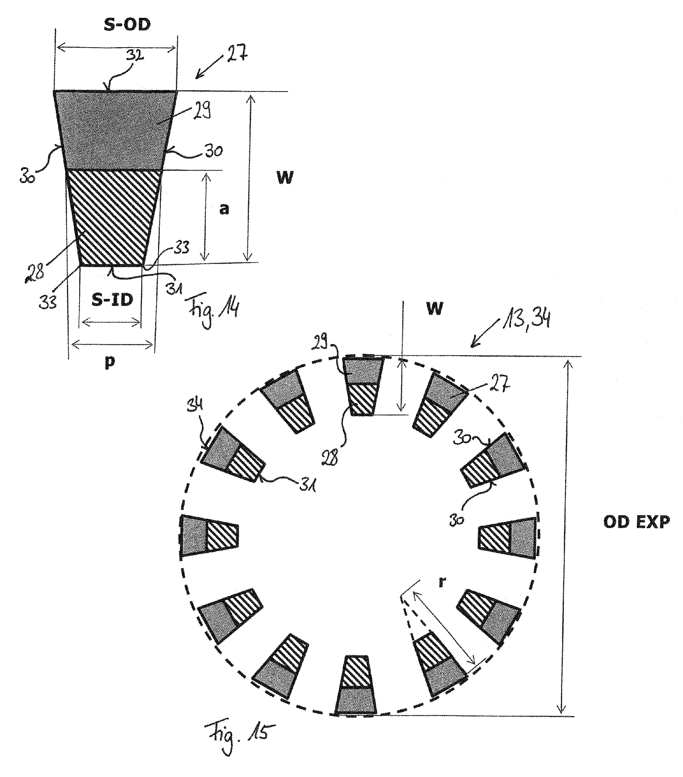

Fig. 14 zeigt einen Querschnitt durch den Funktionsabschnitt 13 eines erfindungsgemäßen medizinischen Instruments, das aus einem Verbundmaterial gebildet ist. Das Verbundmaterial umfasst zwei oder mehr Materialkomponenten 28, 29, die sich hinsichtlich Röntgensichtbarkeit und/oder elektrischer Leitfähigkeit unterscheiden. Konkret ist vorgesehen, dass der expandierbare Funktionsabschnitt 13 aus einem einzigen Draht hergestellt ist, der als Verbunddraht ausgebildet ist, also ein Verbundmaterial aufweist. Insbesondere kann der Draht einen Kerndraht aus einer ersten Materialkomponente 28 und eine Umhüllung aus einer zweiten Materialkomponente 29 aufweisen. Die erste Materialkomponente 28 weist vorzugsweise eine höhere Röntgensichtbarkeit als die zweite Materialkomponente 29 auf. Auf diese Weise ergibt sich ein zylindrisches Element 11, das mehrschichtig aufgebaut ist, wobei die einzelnen Schichten konzentrisch bzw. koaxial zueinander angeordnet sind.

Fig. 15 zeigt im Detail einen Querschnitt durch einen Steg 27 der Umfangswandung gemäß Fig. 14. Es ist gut erkennbar, dass der Steg 27 eine erste Materialkomponente 28 und eine zweite Materialkomponente 29 aufweist. Die erste Materialkomponente 28 ist radial innerhalb der zweiten Materialkomponente 29 angeordnet. Insbesondere ist die erste Materialkomponente 28 koaxial innerhalb der zweiten Materialkomponente 29 angeordnet. Ferner ist aus Fig. 15 gut erkennbar, dass die Seitenflächen 30 des Stegs 27 zueinander konvergieren und auf einem Innenumfang des Funktionsabschnitts 13 durch eine Innenfläche 31 miteinander verbunden sind. Mit anderen Worten bildet der Steg 27 einen kuchenstückartigen Querschnitt mit abgeflachter Spitze, wobei die abgeflachte Spitze durch die Innenfläche 31 gebildet ist. Aus Gründen der Übersichtlichkeit ist in der schematischen Darstellung des Stegs 27 gemäß Fig. 15 die Abrundung der Kanten nicht dargestellt.

Mit Bezug auf die Figuren 14 und 15 werden nachfolgend einige bevorzugte Dimensionen des Funktionsabschnitts 13 angegeben, die generell für alle Ausführungsbeispiele der Erfindung gelten:
So weist der Funktionsabschnitt 13 im expandierten Zustand vorzugsweise einen Außendurchmesser OD EXP auf, der zwischen 2,5 mm und 8 mm, insbesondere zwischen 3 mm und 7 mm, insbesondere zwischen 3,5 mm und 6 mm, insbesondere 4,5 mm beträgt. Das Verhältnis zwischen der Wandstärke W des Funktionsabschnitts 13 bzw. der Stegdicke und dem Außendurchmesser OD EXP des Funktionsabschnitts 13 im expandierten Zustand, d.h. das Verhältnis W/D, beträgt vorzugsweise zwischen 0,00625 und 0,04, insbesondere zwischen 0,008 und 0,03, insbesondere zwischen 0,01 und 0,02.

Der Abstand zwischen einer Außenfläche 32 des Stegs 27 und einem Schnittpunkt der Seitenflächen 30 wird als Schnitttiefe r bezeichnet. Die Schnitttiefe r entspricht dem Verhältnis zwischen dem halben Durchmesser eines Katheters, durch welchen das Instrument 10 geschoben werden kann, und dem Außendurchmesser des Funktionsabschnitts 13 im expandierten Zustand. Das Verhältnis zwischen der Schnitttiefe r und dem Außendurchmesser OD EXP des Funktionsabschnitts 13 im expandierten Zustand, d.h. das Verhältnis r/D, beträgt vorzugsweise zwischen 0,025 und 0,08, insbesondere zwischen 0,03 und 0,06, insbesondere zwischen 0,04 und 0,05. Der Außendurchmesser des Drahts, aus dem durch Laserschneiden das Instrument 10, insbesondere das Zylindrische Element 11, hergestellt wird, entspricht im ungeschnittenen Zustand der doppelten Schnitttiefe r. Vorzugsweise beträgt daher der Außendurchmesser des Drahts, der zur Herstellung des Instruments 10 genutzt wird, (2 ·r), insbesondere zwischen 0,2 mm und 0,5 mm, insbesondere zwischen 0,3 mm und 0,4 mm.

Anhand der Schnittansicht gemäß Fig. 15 werden im Folgenden einige Dimensionen angegeben. Dabei wird darauf hingewiesen, dass sich die Dimensionsangaben auf den Steg 27 nach dem Schritt des Elektropolierens beziehen. Mit anderen Worten weist der Steg 27 im Bereich seiner Kanten jeweils eine Abrundung auf. Die Abrundung beträgt vorzugsweise wenigstens 5 µm, insbesondere wenigstens 10 µm, insbesondere wenigstens 15 µm, jedenfalls jedoch maximal 30 µm. Fig. 12 zeigt den Steg 27 mit einer Innenfläche 31. Es ist allerdings auch möglich, dass der Steg 27 eine Innenkante 33 aufweist, die die beiden Seitenflächen 30 direkt miteinander verbindet. Mit anderen Worten kann die Innenkante 33 vollständig abgerundet sein, so dass die Innenkante 33 mit einer Innenfläche 31 verschliffen ist.

Der Steg 27 gemäß Fig. 15 weist zwei Materialkomponenten 28, 29 auf. Diese bilden jeweils eine Materialschicht, wobei die Schichtdicke a der ersten Materialkomponente 28 vorzugsweise zwischen 5 µm und 20 µm, insbesondere zwischen 6 µm und 15 µm, insbesondere zwischen 8 µm und 12 µm, vorzugsweise 10 µm, beträgt. Die gesamte Stegdicke bzw. Wandstärke W beträgt vorzugsweise zwischen 30 µm und 100 µm, insbesondere zwischen 40 µm und 80 µm, insbesondere zwischen 50 µm und 70 µm, vorzugsweise 60 µm.

Das Verhältnis zwischen der Schichtdicke a der ersten Materialkomponente 28 und der Wandstärke W, also das Verhältnis a/W, beträgt vorzugsweise zwischen 5 % und 40 %, insbesondere zwischen 10 % und 30 %, insbesondere zwischen 15 % und 25 %, vorzugsweise 20 %. Die vorgenannten Werte gelten insbesondere, wenn die erste Materialkomponente 28 ein röntgensichtbares Material aufweist bzw. eine höhere Röntgensichtbarkeit als die zweite Materialkomponente 29 umfasst. In dem dargestellten Ausführungsbeispiel ist die erste Materialkomponente 28 auf einem Innenumfang des Funktionsabschnitts 13 angeordnet. Es ist auch möglich, dass sich die erste Materialkomponente, vorzugsweise einschließlich der zuvor genannten Dimensionen der Schichtdicke a, auf einem Außenumfang des Funktionsabschnitts 13 befindet. Die Anordnung der ersten Materialkomponente 28 auf dem Außenumfang ist insbesondere bevorzugt, wenn die erste Materialkomponente 28 eine höhere elektrische Leitfähigkeit als die zweite Materialkomponente 29 aufweist. Ferner kann die erste Materialkomponente 28 eine Zwischenschicht bilden, die zwischen zwei weiteren Schichten eingebettet ist. Wenn die erste Materialkomponente 28 eine verbesserte elektrische Leitfähigkeit aufweist, kann die Zwischenschicht insbesondere ein elektrisch isolierendes Material umfassen. Die erste Materialkomponente 28 ist dann vorzugsweise auf dem Außenumfang angeordnet und durch die Zwischenschicht von der zweiten Materialkomponente 29 getrennt, die auf dem Innenumfang angeordnet ist und eine tragende Funktion übernimmt.

Vorzugsweise weist der Draht, aus dem der Funktionabschnitt 13 geschnitten wird, ein Volumenverhältnis zwischen der ersten Materialkomponente 28 und der zweiten Materialkomponente 29 auf, das zwischen 10 % und 50 %, insbesondere zwischen 12 % und 40 %, insbesondere zwischen 15 % und 30 %, vorzugsweise 20 % beträgt. In diesem Fall ist die erste Materialkomponente 28 mit einer höheren Röntgensichtbarkeit als die zweite Materialkomponente 29 ausgestattet.

Die Stegbreite S-OD am Außendurchmesser des Funktionsabschnitts 13 beträgt vorzugsweise zwischen 20 µm und 75 µm, insbesondere zwischen 22 µm und 50 µm, insbesondere zwischen 25 µm und 45 µm, insbesondere zwischen 30 µm und 40 µm. Die Stegbreite S-ID am Innendurchmesser des Funktionsabschnitts 13 beträgt vorzugsweise zwischen 10 µm und 50 µm, insbesondere zwischen 15 µm und 30 µm, insbesondere zwischen 18 µm und 25 µm. Die vorgenannten Werte entsprechen vorzugsweise der maximal möglichen Rundung am Innenumfang der Umfangswandung 10. Das Verhältnis zwischen der Stegbreite S-ID am Innendurchmesser des Funktionsabschnitts 13 zur Stegbreite S-OD an der Außenfläche 12 des Funktionsabschnitts 13, also das Verhältnis S-ID/S-OD, ist vorzugsweise kleiner als 0,8, insbesondere kleiner als 0,6, insbesondere kleiner als 0,4, insbesondere kleiner als 0,3, aber größer als 0,1.

Fig. 15 zeigt außerdem eine Zwischenbreite p, die der Stegbreite im Bereich des Übergangs zwischen den zwei Materialkomponenten 28, 29 entspricht. Die Zwischenbreite p beträgt vorzugsweise zwischen 20 µm und 60 µm, insbesondere zwischen 25µm und 35 µm.

Das Verhältnis p/S-OD zwischen der Zwischenbreite p und der Stegbreite S-OD am Außendurchmesser des Funktionsabschnitts 13 ist vorzugsweise kleiner als 0,8, insbesondere kleiner als 0,7, insbesondere kleiner als 0,6, insbesondere kleiner als 0,5, jedoch größer als 0,2.

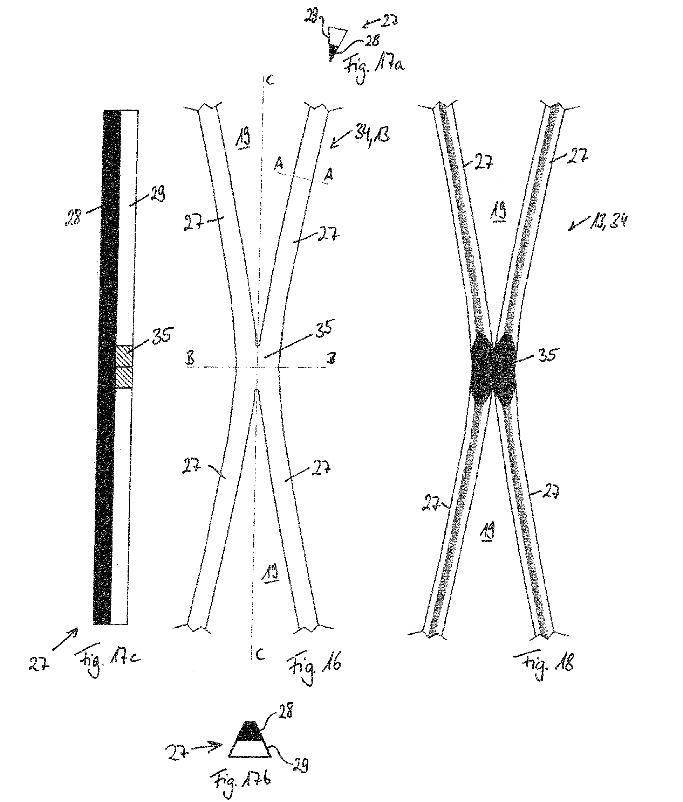

In Fig. 16 ist eine Draufsicht auf ein Teil einer Gitterstruktur 34 gezeigt, die nach dem erfindungsgemäßen Verfahren hergestellt ist. Die Gitterstruktur 34 weist mehrere Stege 27 auf, die Zellen 19 begrenzen. Die Stege 27 weisen eine kuchenstückartige Querschnittsform auf.

So zeigt Fig. 17a beispielsweise einen Querschnitt des Stegs 27 entlang der Linie A-A in Fig. 16. Es ist erkennbar, dass der Steg 27 zwei Materialkomponenten 28, 29 aufweist, wobei eine erste Materialkomponente 28 mit einer relativ höheren Röntgensichtbarkeit radial innerhalb der zweiten Materialkomponente 29 mit der relativ niedrigeren Röntgensichtbarkeit angeordnet ist. Im Wesentlichen ist die Gitterstruktur 34 bzw. der Funktionsabschnitt 13 also zweischichtig ausgebildet, wobei eine radial innere Schicht eine höhere Röntgensichtbarkeit als eine radial äußere Schicht aufweist.

Fig. 17b zeigt eine Schnittansicht durch die Gitterstruktur 34 entlang der Linie B-B in Fig. 13. Bedingt durch das Zusammentreffen mehrerer Stege 27 im Bereich einer Verbindungsstelle 35 der Gitterstruktur 21 zeigt die Querschnittsansicht gemäß Fig. 17b eine abgeflachte Spitze des an sich kuchenstückartigen Querschnitts. Fig. 17c zeigt eine Längsschnittansicht des Funktionsabschnitts 13 entlang der Linie C-C in Fig. 16. Es ist gut erkennbar, dass die einzelnen Stege 11 vollständig aus zwei Materialkomponenten 28, 29 gebildet sind. Die beiden Materialkomponenten 28, 29 bilden gemeinsam ein Verbundmaterial.

Fig. 18 zeigt eine Röntgenaufnahme des Gitterstrukturausschnitts gemäß Fig. 16. Es ist gut erkennbar, dass die Röntgensichtbarkeit insbesondere durch das Materialvolumen der ersten Materialkomponente 28 beeinflusst wird. So ist im Bereich der Stege 27 wegen der kuchenstückartigen Querschnittsform ein geringeres Materialvolumen der ersten Materialkomponente 28 vorhanden als im Bereich der Verbindungsstelle 35. Die Verbindungsstelle 35 ist daher unter Röntgenstrahlung besser sichtbar als die Stege 27. Die Röntgensichtbarkeit kann also über das gesamte zylindrische Element 11 bzw. das gesamte Implantat 10 unterschiedlich ausgeprägt sein.

Die nachfolgende Beschreibung allgemeiner Merkmale der Erfindung gilt für alle Ausführungsbeispiele:
Im Allgemeinen wird im Rahmen der vorliegenden Anmeldung ein Instrument beschrieben, das wenigstens einen Steg 27 aufweist, der eine kuchenstückförmige Querschnittsform aufweist. Dabei ist eine Spitze der kuchenstückartigen Querschnittsform nach innen gerichtet. Mit anderen Worten konvergieren die Seitenflächen 30 des Stegs 27 radial nach innen zu einer Längsachse des Funktionsabschnitts 13. Vorzugsweise kann der Steg wenigstens zwei Materialkomponenten 28, 29 aufweisen, wobei eine erste Materialkomponente 28 eine höhere Röntgensichtbarkeit als eine zweite Materialkomponente 29 aufweist. Es ist auch möglich, dass die erste Materialkomponente 28 eine höhere elektrische Leitfähigkeit als die zweite Materialkomponente 29 aufweist. Vorzugsweise ist die erste Materialkomponente 28 mit der höheren Röntgensichtbarkeit radial innerhalb der zweiten Materialkomponente 29 angeordnet. Im Allgemeinen kann das Instrument 10 mehrere Stege 27 aufweisen. Die Stege 27 bilden gemeinsam den Funktionsabschnitt 13, insbesondere eine Gitterstruktur 34. Die Gitterstruktur 34 ist radial expandierbar. Vorzugsweise erstrecken sich die Stege 27 hauptsächlich in Längsrichtung der Gitterstruktur 34 oder helixförmig um eine Längsachse des Funktionsabschnitts 13. Eine derartige Gestaltung des Funktionsabschnitts 13 ist insbesondere für Blutfilter und Thrombectomiedevices geeignet. Die Stege 27 können im Allgemeinen eine Gitterstruktur 34 bilden, was insbesondere für den Einsatz des medizinischen Instruments als Stent, Flowdiverter oder Thrombectomiedevice vorteilhaft ist.

Ferner kann ein Teil des medizinischen Instruments 10 unstrukturiert sein, also nicht durch Laserschneiden bearbeitet sein. Dieser Teil des Instruments 10 bildet vorzugsweise den Führungsabschnitt 14, der im Wesentlichen durchmesserstabil ausgebildet ist. Auf diese Weise kann ein distaler Endabschnitt 12, der als expandierbarer Funktionsabschnitt 13 ausgebildet ist, an den Behandlungsort geführt werden. Ein medizinisches Instrument 10, das zum temporären Einsatz in Blutgefäßen vorgesehen ist und einen radial expandierbaren distalen Endabschnitt 12 und einen proximalen, durchmesserstabilen Führungsabschnitt 13 aufweist, der als Transportelement 20 dient, kann insbesondere als Thrombectomiedevice, Blutfilter oder auch als Elektrode zur Stimulation oder Ablation eingesetzt werden.

Es ist ferner möglich, mehrere, expandierbare Funktionsabschnitte 13 miteinander zu koppeln. So kann ein medizinisches Instrument 10 gebildet werden, das aus mehreren, längsaxial hintereinander angeordneten, expandierbaren Funktionsabschnitten 13 gebildet ist. Zwischen den einzelnen Funktionsabschnitten 13 können gelenkartige Elemente angeordnet sein. Die gelenkartigen Elemente können durch durchmesserstabile Abschnitte gebildet sein. Vorzugsweise ist das gesamte medizinische Instrument monolithisch ausgebildet, wobei die expandierbaren Funktionsabschnitte 13 bzw. Gitterstrukturen 34 durch Strukturierung eines einzelnen Ausgangsdrahts, d.h. des zylindrischen Elements11, hergestellt sind.

Das erfindungsgemäße Instrument ist vorzugsweise über einen Katheter in den menschlichen Körper einführbar, dessen Innendurchmesser höchstens 0,7 mm, insbesondere höchstens 0,51 mm, insbesondere höchstens 0,42 mm, beträgt.

Die Erfindung ist auf medizinische Vorrichtungen anwendbar, die temporär in einem Körperhohlraum eingesetzt werden. Insbesondere ist die Erfindung auch auf Thrombektomiedevices oder Blutfilter anwendbar, die einen expandierbaren Funktionsabschnitt 13 aufweisen. Der expandierbare Funktionsabschnitt 13 ist fest, insbesondere monolithisch, mit einem Transportelement 20, beispielweise einem Führungsdraht, verbunden. Der expandierbare Funktionsabschnitt 13 bildet vorzugsweise einen distalen Endabschnitt 12 des Transportelements 20. Das Transportelement 20 kann als Volldraht oder als Hohldraht ausgebildet sein. Insgesamt kann das gesamte medizinische Instrument 10 monolithisch aus einem Voll- oder Hohldraht gebildet sein. Der Voll- oder Hohldraht kann aus einem Verbundmaterial bestehen, das wenigstens zwei unterschiedliche Materialkomponenten 28, 29 enthält. Insbesondere kann ein Kerndraht mit einer Umhüllung vorgesehen sein, wobei der Kerndraht eine erste Materialkomponente 28 und die Umhüllung eine zweite Materialkomponente 29 enthält. Die erste Materialkomponente 28 kann eine höhere Röntgensichtbarkeit und/oder elektrische Leitfähigkeit als die zweite Materialkomponente 29 aufweisen.

Die Gitterstruktur 34 weist Stege 27 und Zellen 19 auf, wobei die Stege 27 die Zellen 19 vollständig begrenzen. Die Anzahl der Zellen 19 in Umfangsrichtung der Gitterstruktur 34 kann unterschiedlich sein. Insbesondere kann die Zellenanzahl je nach Verwendungszweck des Instruments 10 eingestellt werden.

Bei der Verwendung der Gitterstruktur 34 als Flow Diverter, vorzugsweise für die Behandlung intrakranieller Aneurysmen, kann die Anzahl der Zellen 19 in Umfangsrichtung zwischen 6 und 24, insbesondere zwischen 9 und 18, vorzugsweise 12, betragen.

Für den Einsatz der Gitterstruktur 34 als Blutfilter oder Elektrode hat sich eine Zellenanzahl zwischen 4 und 16, insbesondere zwischen 6 und 12, vorzugsweise 9, als vorteilhaft erwiesen. Für denselben Einsatzzweck kann auch ein Funktionsabschnitt 13 vorgesehen sein, die längsaxial und/oder helixförmig sich um die Längsachse des Funktionsabschnitts 13 erstreckende Stege 27 aufweist. Dabei bildet der Funktionsabschnitt 13 vorzugsweise zwischen 4 und 24, insbesondere zwischen 6 und 18, insbesondere zwischen 9 und 12, Zellen in Umfangsrichtung aus.

### Bezugszeichenliste

- 10: Instrument
- 11: Zylindrisches Element
- 12: Endabschnitt
- 13: Funktionsabschnitt
- 14: Führungsabschnitt
- 15: Längsschnitt
- 16: Distales Ende
- 17: Einschnitt
- 18: Greifarm
- 19: Zelle
- 20: Transportelement
- 21: Proximaler Bereich
- 22: Distaler Bereich
- 23: Proximales Ende
- 24: Distales Ende
- 25: Flexionselement
- 26: Ring
- 27: Steg
- 27a: Stegelement
- 28: erste Materialkomponente
- 29: zweite Materialkomponente
- 30: Seitenfläche
- 31: Innenfläche
- 32: Außenfläche
- 33: Innenkante
- 34: Gitterstruktur
- 35: Verbindungsstelle

## Patentansprüche

1. Medizinisches Instrument für intravaskuläre Eingriffe mit einem zylindrischen Element (11), das durch einen Katheter in einen Körperhohlraum einführbar ist, wobei das zylindrische Element (11) einen distalen Endabschnitt (12) mit einem radial expandierbaren Funktionsabschnitt (13) und einem durchmesserstabilen, zylindrischen Führungsabschnitt (14) aufweist, der proximal zum Funktionsabschnitt (13) angeordnet ist,
**dadurch gekennzeichnet,dass**
der distale Endabschnitt (12) zur Bildung des Funktionsabschnitts (13) strukturiert ist derart, dass der Funktionsabschnitt (13) wenigstens einen Steg (27) aufweist, der eine einstückige Verlängerung des Führungsabschnitts (14) bildet.

2. Instrument nach Anspruch 1,
**dadurch gekennzeichnet, dass**
der Funktionsabschnitt (13) eine zumindest abschnittsweise röhrchenförmige Wandung aufweist, die durch den Steg, insbesondere durch mehrere Stege (27) gebildet ist und/oder dass der Funktionsabschnitt (13) mehrere über den Umfang verteilte Längsschnitte (15) zur Bildung des wenigstens einen Steges (27), insbesondere zur Bildung mehrerer Stege (27) aufweist.

3. Instrument nach Anspruch 2,
**dadurch gekennzeichnet, dass**
die Längsschnitte (15) in Längsrichtung zueinander versetzt sind derart, dass der Funktionsabschnitt (13) eine netzartige Struktur aufweist, oder dass sich die Längsschnitte (15) bis zum distalen Ende (24) des Funktionsabschnitts (13) erstrecken derart, dass wenigstens zwei Stege (27) einen Greifer bilden.

4. Instrument nach einem der vorhergehenden Ansprüche,
**dadurch gekennzeichnet, dass**
der Führungsabschnitt (14) mehrere in Umfangsrichtung orientierte Einschnitte (17) aufweist derart, dass der Führungsabschnitt (14) in einem distalen Bereich (22) eine höhere Biegeflexibilität als in einem proximalen Bereich (21) aufweist.

5. Instrument nach Anspruch 4,
**dadurch gekennzeichnet, dass**
die Breite und/oder Tiefe der Einschnitte (17) vom distalen Bereich zum proximalen Bereich des Führungsabschnitts (14) abnimmt und/oder der Abstand der Einschnitte (17) vom distalen Bereich (22) zum proximalen Bereich (21) des Führungsabschnitts (14) zunimmt.

6. Instrument nach Anspruch 4 oder 5,
**dadurch gekennzeichnet, dass**
die Einschnitte (17) ringförmig oder helixförmig umlaufende Materialschwächungen und/oder Materialdurchbrüche umfassen, insbesondere dass die Einschnitte (17), insbesondere die Materialdurchbrüche, zumindest bereichsweise um eine Längsachse des Führungsabschnitts (14) verdreht angeordnet sind.

7. Instrument nach einem der vorhergehenden Ansprüche,
**dadurch gekennzeichnet, dass**
das zylindrische Element (11) durch einen Volldraht oder einen Hohldraht gebildet ist.

8. Instrument nach einem der vorhergehenden Ansprüche,
**dadurch gekennzeichnet, dass**
der Führungsabschnitt (14) ein Transportelement (20), insbesondere einen Transportdraht oder ein Transportdrahtsegment, bildet wobei das Transportdrahtsegment mit einem Transportdraht verbunden oder verbindbar ist, der sich bis zu einem proximalen Ende (23) des Instruments (10) erstreckt.

9. Instrument nach Anspruch 8,
**dadurch gekennzeichnet, dass**
das Transportelement (20) wenigstens ein Flexionselement (25), insbesondere zur kontinuierlichen oder schrittweisen Steigerung der Biegeflexibilität aufweist.

10. Instrument nach einem der vorhergehenden Ansprüche,
**dadurch gekennzeichnet,dass**
das zylindrische Element (11) aus einem Verbundmaterial gebildet ist, das wenigstens zwei Materialkomponenten aufweist.

11. Instrument nach Anspruch 10,
**dadurch gekennzeichnet, dass**
eine erste Materialkomponente eine höhere Röntgensichtbarkeit und/oder elektrische Leitfähigkeit als eine zweite Materialkomponente aufweist.

12. Instrument nach Anspruch 11,
**dadurch gekennzeichnet, dass**
die erste Materialkomponente koaxial zur und radial innerhalb der zweiten Materialkomponente angeordnet ist.

13. Verfahren zur Herstellung eines medizinischen Instruments (10) für intravaskuläre Eingriffe aus einem zylindrischen Element (11), das durch einen Katheter in einen Körperhohlraum einführbar ist, wobei ein distaler Endabschnitt (12) des zylindrischen Elements (11) durch einen materialabtragenden Bearbeitungsschritt strukturiert wird derart, dass ein radial expandierbarer Funktionsabschnitt (13) des Instruments (10) einstückig mit einem proximal angeordneten Führungsabschnitt (14) ausgebildet wird.

14. Verfahren zur Herstellung eines medizinischen Instruments (10) für intravaskuläre Eingriffe aus einem zylindrischen Element (11), das durch einen Katheter in einen Körperhohlraum einführbar ist, wobei ein distaler Endabschnitt (12) des zylindrischen Elements (11) durch einen materialabtragenden Bearbeitungsschritt strukturiert wird derart, dass aus einem Vollmaterial, insbesondere einem Volldraht, ein radial expandierbares Funktionselement, insbesondere ein radial expandierbarer Funktionsabschnitt (13), des Instruments (10) gebildet wird.

15. Verfahren nach Anspruch 13 oder 14,
**dadurch gekennzeichnet, dass**
der materialabtragende Bearbeitungsschritt Laserschneiden umfasst.
